(19)

Europäisches
Patentamt
European
Patent Office
Office européen
des brevets

(11) **EP 2 505 572 A1**

(12) **EUROPEAN PATENT APPLICATION**

(43) Date of publication:
**03.10.2012 Bulletin 2012/40**

(51) Int Cl.:
***C07B 59/00*** (2006.01)     ***C12Q 1/48*** (2006.01)
***G01N 33/58*** (2006.01)

(21) Application number: **11002746.3**

(22) Date of filing: **01.04.2011**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**

(71) Applicant: **Max-Planck-Gesellschaft zur
Förderung der
Wissenschaften e.V.
80539 München (DE)**

(72) Inventors:
• **Rauh, Daniel
  67549 Worms (DE)**
• **Simard, Jeffrey Raymond
  Salem
  MA 01970 (US)**

(74) Representative: **Vossius & Partner
Siebertstrasse 4
81675 München (DE)**

(54) **Kinases labeled in the C helix for the screening of inhibitors**

(57)     The present invention relates to a kinase containing exactly one label in the C helix, (a) wherein said label is a fluorophore, a spin label or an isotope label, (a)(i) wherein said fluorophore and said spin label are covalently bound to a thiol group or side chain amino group of an amino acid residue of said C helix, (a)(ii) wherein said isotope label is a moiety comprising one or more atoms which are isotopes with non-zero nuclear spin, said moiety being covalently bound to a thiol group or side chain amino group of an amino acid residue of said C helix, or said isotope label is an amino acid residue of said C helix, said amino acid residue comprising one or more atoms which are isotopes with non-zero nuclear spin, wherein said isotopes with non-zero nuclear spin are enriched as compared to the natural occurrence thereof.

SEQUENCE LISTING

<110>  Max-Planck-Gesellschaft zur Förderung der Wissenschaften e.V.

<120>  Kinases labeled in the C helix for the screening of inhibitors

<130>  T1082 EP

<160>  12

<170>  PatentIn version 3.5

<210>  1
<211>  318
<212>  PRT
<213>  Homo sapiens

<400>  1

Gly Pro Gly Ala Leu Leu Arg Ile Leu Lys Glu Thr Glu Phe Lys Lys
1               5                   10                  15

Ile Lys Val Leu Gly Ser Gly Ala Phe Gly Thr Val Tyr Lys Gly Leu
            20                  25                  30

Trp Ile Pro Glu Gly Glu Lys Val Lys Ile Pro Val Ala Ile Lys Glu
        35                  40                  45

Leu Arg Glu Ala Thr Ser Pro Lys Ala Asn Lys Cys Ile Leu Asp Glu
    50                  55                  60

Ala Tyr Val Met Ala Ser Val Asp Asn Pro His Val Cys Arg Leu Leu
65                  70                  75                  80

Gly Ile Ser Leu Thr Ser Thr Val Gln Leu Ile Thr Gln Leu Met Pro
                85                  90                  95

Phe Gly Ser Leu Leu Asp Tyr Val Arg Glu His Lys Asp Asn Ile Gly
            100                 105                 110

Ser Gln Tyr Leu Leu Asn Trp Cys Val Gln Ile Ala Lys Gly Met Asn
        115                 120                 125

Tyr Leu Glu Asp Arg Arg Leu Val His Arg Asp Leu Ala Ala Arg Asn
    130                 135                 140

Val Leu Val Lys Thr Pro Gln His Val Lys Ile Thr Asp Phe Gly Leu
145                 150                 155                 160

Ala Lys Leu Leu Gly Ala Glu Glu Lys Glu Tyr His Ala Glu Gly Gly
                165                 170                 175

Lys Val Pro Ile Lys Trp Met Ala Leu Glu Ser Ile Leu His Arg Ile
                180                 185                 190

17

```
Tyr Thr His Gln Ser Asp Val Trp Ser Tyr Gly Val Thr Val Trp Glu
        195                 200                 205

Leu Met Thr Phe Gly Ser Lys Pro Tyr Asp Gly Ile Pro Ala Ser Glu
        210                 215                 220

Ile Ser Ser Ile Leu Glu Lys Gly Glu Arg Leu Pro Gln Pro Pro Ile
225                 230                 235                 240

Ser Thr Ile Asp Val Tyr Met Ile Met Val Lys Cys Trp Met Ile Asp
                245                 250                 255

Ala Asp Ser Arg Pro Lys Phe Arg Glu Leu Ile Ile Glu Phe Ser Lys
                260                 265                 270

Met Ala Arg Asp Pro Gln Arg Tyr Leu Val Ile Gln Gly Asp Glu Arg
                275                 280                 285

Met His Leu Pro Ser Pro Thr Asp Ser Asn Phe Tyr Arg Ala Leu Met
        290                 295                 300

Asp Glu Glu Asp Met Asp Asp Val Val Asp Ala Asp Glu Tyr
305                 310                 315
```

```
<210>  2
<211>  536
<212>  PRT
<213>  Homo sapiens

<400>  2
```

```
Met Gly Ser Asn Lys Ser Lys Pro Lys Asp Ala Ser Gln Arg Arg Arg
1               5                   10                  15

Ser Leu Glu Pro Ala Glu Asn Val His Gly Ala Gly Gly Gly Ala Phe
                20                  25                  30

Pro Ala Ser Gln Thr Pro Ser Lys Pro Ala Ser Ala Asp Gly His Arg
                35                  40                  45

Gly Pro Ser Ala Ala Phe Ala Pro Ala Ala Ala Glu Pro Lys Leu Phe
        50                  55                  60

Gly Gly Phe Asn Ser Ser Asp Thr Val Thr Ser Pro Gln Arg Ala Gly
65                  70                  75                  80

Pro Leu Ala Gly Gly Val Thr Thr Phe Val Ala Leu Tyr Asp Tyr Glu
                85                  90                  95

Ser Arg Thr Glu Thr Asp Leu Ser Phe Lys Lys Gly Glu Arg Leu Gln
                100                 105                 110

Ile Val Asn Asn Thr Glu Gly Asp Trp Trp Leu Ala His Ser Leu Ser
```

115 120 125

Thr Gly Gln Thr Gly Tyr Ile Pro Ser Asn Tyr Val Ala Pro Ser Asp
130 135 140

Ser Ile Gln Ala Glu Glu Trp Tyr Phe Gly Lys Ile Thr Arg Arg Glu
145 150 155 160

Ser Glu Arg Leu Leu Leu Asn Ala Glu Asn Pro Arg Gly Thr Phe Leu
165 170 175

Val Arg Glu Ser Glu Thr Thr Lys Gly Ala Tyr Cys Leu Ser Val Ser
180 185 190

Asp Phe Asp Asn Ala Lys Gly Leu Asn Val Lys His Tyr Lys Ile Arg
195 200 205

Lys Leu Asp Ser Gly Gly Phe Tyr Ile Thr Ser Arg Thr Gln Phe Asn
210 215 220

Ser Leu Gln Gln Leu Val Ala Tyr Tyr Ser Lys His Ala Asp Gly Leu
225 230 235 240

Cys His Arg Leu Thr Thr Val Cys Pro Thr Ser Lys Pro Gln Thr Gln
245 250 255

Gly Leu Ala Lys Asp Ala Trp Glu Ile Pro Arg Glu Ser Leu Arg Leu
260 265 270

Glu Val Lys Leu Gly Gln Gly Cys Phe Gly Glu Val Trp Met Gly Thr
275 280 285

Trp Asn Gly Thr Thr Arg Val Ala Ile Lys Thr Leu Lys Pro Gly Thr
290 295 300

Met Ser Pro Glu Ala Phe Leu Gln Glu Ala Gln Val Met Lys Lys Leu
305 310 315 320

Arg His Glu Lys Leu Val Gln Leu Tyr Ala Val Val Ser Glu Glu Pro
325 330 335

Ile Tyr Ile Val Thr Glu Tyr Met Ser Lys Gly Ser Leu Leu Asp Phe
340 345 350

Leu Lys Gly Glu Thr Gly Lys Tyr Leu Arg Leu Pro Gln Leu Val Asp
355 360 365

Met Ala Ala Gln Ile Ala Ser Gly Met Ala Tyr Val Glu Arg Met Asn
370 375 380

Tyr Val His Arg Asp Leu Arg Ala Ala Asn Ile Leu Val Gly Glu Asn

385                    390                    395                    400

Leu Val Cys Lys Val Ala Asp Phe Gly Leu Ala Arg Leu Ile Glu Asp
            405             410             415

Asn Glu Tyr Thr Ala Arg Gln Gly Ala Lys Phe Pro Ile Lys Trp Thr
            420             425             430

Ala Pro Glu Ala Ala Leu Tyr Gly Arg Phe Thr Ile Lys Ser Asp Val
            435             440             445

Trp Ser Phe Gly Ile Leu Leu Thr Glu Leu Thr Thr Lys Gly Arg Val
    450             455             460

Pro Tyr Pro Gly Met Val Asn Arg Glu Val Leu Asp Gln Val Glu Arg
465             470             475             480

Gly Tyr Arg Met Pro Cys Pro Pro Glu Cys Pro Glu Ser Leu His Asp
            485             490             495

Leu Met Cys Gln Cys Trp Arg Lys Glu Pro Glu Glu Arg Pro Thr Phe
            500             505             510

Glu Tyr Leu Gln Ala Phe Leu Glu Asp Tyr Phe Thr Ser Thr Glu Pro
            515             520             525

Gln Tyr Gln Pro Gly Glu Asn Leu
    530             535

<210>   3
<211>   1225
<212>   PRT
<213>   Homo sapiens

<400>   3

Met Lys Leu Arg Leu Pro Ala Ser Pro Glu Thr His Leu Asp Met Leu
1               5               10              15

Arg His Leu Tyr Gln Gly Cys Gln Val Val Gln Gly Asn Leu Glu Leu
            20              25              30

Thr Tyr Leu Pro Thr Asn Ala Ser Leu Ser Phe Leu Gln Asp Ile Gln
        35              40              45

Glu Val Gln Gly Tyr Val Leu Ile Ala His Asn Gln Val Arg Gln Val
    50              55              60

Pro Leu Gln Arg Leu Arg Ile Val Arg Gly Thr Gln Leu Phe Glu Asp
65              70              75              80

Asn Tyr Ala Leu Ala Val Leu Asp Asn Gly Asp Pro Leu Asn Asn Thr
            85              90              95

EP 2 505 572 A1

Thr Pro Val Thr Gly Ala Ser Pro Gly Gly Leu Arg Glu Leu Gln Leu
100 105 110

Arg Ser Leu Thr Glu Ile Leu Lys Gly Gly Val Leu Ile Gln Arg Asn
115 120 125

Pro Gln Leu Cys Tyr Gln Asp Thr Ile Leu Trp Lys Asp Ile Phe His
130 135 140

Lys Asn Asn Gln Leu Ala Leu Thr Leu Ile Asp Thr Asn Arg Ser Arg
145 150 155 160

Ala Cys His Pro Cys Ser Pro Met Cys Lys Gly Ser Arg Cys Trp Gly
165 170 175

Glu Ser Ser Glu Asp Cys Gln Ser Leu Thr Arg Thr Val Cys Ala Gly
180 185 190

Gly Cys Ala Arg Cys Lys Gly Pro Leu Pro Thr Asp Cys Cys His Glu
195 200 205

Gln Cys Ala Ala Gly Cys Thr Gly Pro Lys His Ser Asp Cys Leu Ala
210 215 220

Cys Leu His Phe Asn His Ser Gly Ile Cys Glu Leu His Cys Pro Ala
225 230 235 240

Leu Val Thr Tyr Asn Thr Asp Thr Phe Glu Ser Met Pro Asn Pro Glu
245 250 255

Gly Arg Tyr Thr Phe Gly Ala Ser Cys Val Thr Ala Cys Pro Tyr Asn
260 265 270

Tyr Leu Ser Thr Asp Val Gly Ser Cys Thr Leu Val Cys Pro Leu His
275 280 285

Asn Gln Glu Val Thr Ala Glu Asp Gly Thr Gln Arg Cys Glu Lys Cys
290 295 300

Ser Lys Pro Cys Ala Arg Val Cys Tyr Gly Leu Gly Met Glu His Leu
305 310 315 320

Arg Glu Val Arg Ala Val Thr Ser Ala Asn Ile Gln Glu Phe Ala Gly
325 330 335

Cys Lys Lys Ile Phe Gly Ser Leu Ala Phe Leu Pro Glu Ser Phe Asp
340 345 350

Gly Asp Pro Ala Ser Asn Thr Ala Pro Leu Gln Pro Glu Gln Leu Gln
355 360 365

21

```
Val Phe Glu Thr Leu Glu Glu Ile Thr Gly Tyr Leu Tyr Ile Ser Ala
    370             375             380

Trp Pro Asp Ser Leu Pro Asp Leu Ser Val Phe Gln Asn Leu Gln Val
385             390             395             400

Ile Arg Gly Arg Ile Leu His Asn Gly Ala Tyr Ser Leu Thr Leu Gln
            405             410             415

Gly Leu Gly Ile Ser Trp Leu Gly Leu Arg Ser Leu Arg Glu Leu Gly
            420             425             430

Ser Gly Leu Ala Leu Ile His His Asn Thr His Leu Cys Phe Val His
        435             440             445

Thr Val Pro Trp Asp Gln Leu Phe Arg Asn Pro His Gln Ala Leu Leu
    450             455             460

His Thr Ala Asn Arg Pro Glu Asp Glu Cys Val Gly Glu Gly Leu Ala
465             470             475             480

Cys His Gln Leu Cys Ala Arg Gly His Cys Trp Gly Pro Gly Pro Thr
            485             490             495

Gln Cys Val Asn Cys Ser Gln Phe Leu Arg Gly Gln Glu Cys Val Glu
            500             505             510

Glu Cys Arg Val Leu Gln Gly Leu Pro Arg Glu Tyr Val Asn Ala Arg
        515             520             525

His Cys Leu Pro Cys His Pro Glu Cys Gln Pro Gln Asn Gly Ser Val
    530             535             540

Thr Cys Phe Gly Pro Glu Ala Asp Gln Cys Val Ala Cys Ala His Tyr
545             550             555             560

Lys Asp Pro Pro Phe Cys Val Ala Arg Cys Pro Ser Gly Val Lys Pro
            565             570             575

Asp Leu Ser Tyr Met Pro Ile Trp Lys Phe Pro Asp Glu Glu Gly Ala
            580             585             590

Cys Gln Pro Cys Pro Ile Asn Cys Thr His Ser Cys Val Asp Leu Asp
        595             600             605

Asp Lys Gly Cys Pro Ala Glu Gln Arg Ala Ser Pro Leu Thr Ser Ile
    610             615             620

Ile Ser Ala Val Val Gly Ile Leu Leu Val Val Val Leu Gly Val Val
625             630             635             640
```

Phe Gly Ile Leu Ile Lys Arg Arg Gln Gln Lys Ile Arg Lys Tyr Thr
645 650 655

Met Arg Arg Leu Leu Gln Glu Thr Glu Leu Val Glu Pro Leu Thr Pro
660 665 670

Ser Gly Ala Met Pro Asn Gln Ala Gln Met Arg Ile Leu Lys Glu Thr
675 680 685

Glu Leu Arg Lys Val Lys Val Leu Gly Ser Gly Ala Phe Gly Thr Val
690 695 700

Tyr Lys Gly Ile Trp Ile Pro Asp Gly Glu Asn Val Lys Ile Pro Val
705 710 715 720

Ala Ile Lys Val Leu Arg Glu Asn Thr Ser Pro Lys Ala Asn Lys Glu
725 730 735

Ile Leu Asp Glu Ala Tyr Val Met Ala Gly Val Gly Ser Pro Tyr Val
740 745 750

Ser Arg Leu Leu Gly Ile Cys Leu Thr Ser Thr Val Gln Leu Val Thr
755 760 765

Gln Leu Met Pro Tyr Gly Cys Leu Leu Asp His Val Arg Glu Asn Arg
770 775 780

Gly Arg Leu Gly Ser Gln Asp Leu Leu Asn Trp Cys Met Gln Ile Ala
785 790 795 800

Lys Gly Met Ser Tyr Leu Glu Asp Val Arg Leu Val His Arg Asp Leu
805 810 815

Ala Ala Arg Asn Val Leu Val Lys Ser Pro Asn His Val Lys Ile Thr
820 825 830

Asp Phe Gly Leu Ala Arg Leu Leu Asp Ile Asp Glu Thr Glu Tyr His
835 840 845

Ala Asp Gly Gly Lys Val Pro Ile Lys Trp Met Ala Leu Glu Ser Ile
850 855 860

Leu Arg Arg Arg Phe Thr His Gln Ser Asp Val Trp Ser Tyr Gly Val
865 870 875 880

Thr Val Trp Glu Leu Met Thr Phe Gly Ala Lys Pro Tyr Asp Gly Ile
885 890 895

Pro Ala Arg Glu Ile Pro Asp Leu Leu Glu Lys Gly Glu Arg Leu Pro
900 905 910

```
Gln Pro Pro Ile Cys Thr Ile Asp Val Tyr Met Ile Met Val Lys Cys
        915                 920             925

Trp Met Ile Asp Ser Glu Cys Arg Pro Arg Phe Arg Glu Leu Val Ser
    930                 935             940

Glu Phe Ser Arg Met Ala Arg Asp Pro Gln Arg Phe Val Val Ile Gln
945             950             955                 960

Asn Glu Asp Leu Gly Pro Ala Ser Pro Leu Asp Ser Thr Phe Tyr Arg
                965             970             975

Ser Leu Leu Glu Asp Asp Asp Met Gly Asp Leu Val Asp Ala Glu Glu
            980             985             990

Tyr Leu Val Pro Gln Gln Gly Phe  Phe Cys Pro Asp Pro  Ala Pro Gly
        995             1000            1005

Ala Gly  Gly Met Val His His  Arg His Arg Ser Ser  Ser Thr Arg
    1010            1015            1020

Ser Gly  Gly Gly Asp Leu Thr  Leu Gly Leu Glu Pro  Ser Glu Glu
    1025            1030            1035

Glu Ala  Pro Arg Ser Pro Leu  Ala Pro Ser Glu Gly  Ala Gly Ser
    1040            1045            1050

Asp Val  Phe Asp Gly Asp Leu  Gly Met Gly Ala Ala  Lys Gly Leu
    1055            1060            1065

Gln Ser  Leu Pro Thr His Asp  Pro Ser Pro Leu Gln  Arg Tyr Ser
    1070            1075            1080

Glu Asp  Pro Thr Val Pro Leu  Pro Ser Glu Thr Asp  Gly Tyr Val
    1085            1090            1095

Ala Pro  Leu Thr Cys Ser Pro  Gln Pro Glu Tyr Val  Asn Gln Pro
    1100            1105            1110

Asp Val  Arg Pro Gln Pro Pro  Ser Pro Arg Glu Gly  Pro Leu Pro
    1115            1120            1125

Ala Ala  Arg Pro Ala Gly Ala  Thr Leu Glu Arg Pro  Lys Thr Leu
    1130            1135            1140

Ser Pro  Gly Lys Asn Gly Val  Val Lys Asp Val Phe  Ala Phe Gly
    1145            1150            1155

Gly Ala  Val Glu Asn Pro Glu  Tyr Leu Thr Pro Gln  Gly Gly Ala
    1160            1165            1170
```

24

Ala Pro Gln Pro His Pro Pro Pro Ala Phe Ser Pro Ala Phe Asp
    1175          1180              1185

Asn Leu Tyr Tyr Trp Asp Gln Asp Pro Pro Glu Arg Gly Ala Pro
    1190          1195              1200

Pro Ser Thr Phe Lys Gly Thr Pro Thr Ala Glu Asn Pro Glu Tyr
    1205          1210              1215

Leu Gly Leu Asp Val Pro Val
    1220          1225

<210>   4
<211>   1130
<212>   PRT
<213>   Homo sapiens

<400>   4

Met Leu Glu Ile Cys Leu Lys Leu Val Gly Cys Lys Ser Lys Lys Gly
1               5               10              15

Leu Ser Ser Ser Ser Ser Cys Tyr Leu Glu Glu Ala Leu Gln Arg Pro
        20              25              30

Val Ala Ser Asp Phe Glu Pro Gln Gly Leu Ser Glu Ala Ala Arg Trp
        35              40              45

Asn Ser Lys Glu Asn Leu Leu Ala Gly Pro Ser Glu Asn Asp Pro Asn
    50              55              60

Leu Phe Val Ala Leu Tyr Asp Phe Val Ala Ser Gly Asp Asn Thr Leu
65              70              75              80

Ser Ile Thr Lys Gly Glu Lys Leu Arg Val Leu Gly Tyr Asn His Asn
            85              90              95

Gly Glu Trp Cys Glu Ala Gln Thr Lys Asn Gly Gln Gly Trp Val Pro
            100             105             110

Ser Asn Tyr Ile Thr Pro Val Asn Ser Leu Glu Lys His Ser Trp Tyr
        115             120             125

His Gly Pro Val Ser Arg Asn Ala Ala Glu Tyr Leu Leu Ser Ser Gly
    130             135             140

Ile Asn Gly Ser Phe Leu Val Arg Glu Ser Glu Ser Ser Pro Gly Gln
145             150             155             160

Arg Ser Ile Ser Leu Arg Tyr Glu Gly Arg Val Tyr His Tyr Arg Ile
            165             170             175

Asn Thr Ala Ser Asp Gly Lys Leu Tyr Val Ser Ser Glu Ser Arg Phe
180                     185                 190

Asn Thr Leu Ala Glu Leu Val His His His Ser Thr Val Ala Asp Gly
195             200                 205

Leu Ile Thr Thr Leu His Tyr Pro Ala Pro Lys Arg Asn Lys Pro Thr
210             215                 220

Val Tyr Gly Val Ser Pro Asn Tyr Asp Lys Trp Glu Met Glu Arg Thr
225             230                 235                 240

Asp Ile Thr Met Lys His Lys Leu Gly Gly Gly Gln Tyr Gly Glu Val
245                 250                 255

Tyr Glu Gly Val Trp Lys Lys Tyr Ser Leu Thr Val Ala Val Lys Thr
260                 265                 270

Leu Lys Glu Asp Thr Met Glu Val Glu Glu Phe Leu Lys Glu Ala Ala
275                 280                 285

Val Met Lys Glu Ile Lys His Pro Asn Leu Val Gln Leu Leu Gly Val
290                 295                 300

Cys Thr Arg Glu Pro Pro Phe Tyr Ile Ile Thr Glu Phe Met Thr Tyr
305                 310                 315                 320

Gly Asn Leu Leu Asp Tyr Leu Arg Glu Cys Asn Arg Gln Glu Val Asn
325                 330                 335

Ala Val Val Leu Leu Tyr Met Ala Thr Gln Ile Ser Ser Ala Met Glu
340                 345                 350

Tyr Leu Glu Lys Lys Asn Phe Ile His Arg Asp Leu Ala Ala Arg Asn
355                 360                 365

Cys Leu Val Gly Glu Asn His Leu Val Lys Val Ala Asp Phe Gly Leu
370                 375                 380

Ser Arg Leu Met Thr Gly Asp Thr Tyr Thr Ala His Ala Gly Ala Lys
385                 390                 395                 400

Phe Pro Ile Lys Trp Thr Ala Pro Glu Ser Leu Ala Tyr Asn Lys Phe
405                 410                 415

Ser Ile Lys Ser Asp Val Trp Ala Phe Gly Val Leu Leu Trp Glu Ile
420                 425                 430

Ala Thr Tyr Gly Met Ser Pro Tyr Pro Gly Ile Asp Leu Ser Gln Val
435                 440                 445

Tyr Glu Leu Leu Glu Lys Asp Tyr Arg Met Glu Arg Pro Glu Gly Cys
    450             455             460

Pro Glu Lys Val Tyr Glu Leu Met Arg Ala Cys Trp Gln Trp Asn Pro
465             470             475             480

Ser Asp Arg Pro Ser Phe Ala Glu Ile His Gln Ala Phe Glu Thr Met
            485             490             495

Phe Gln Glu Ser Ser Ile Ser Asp Glu Val Glu Lys Glu Leu Gly Lys
            500             505             510

Gln Gly Val Arg Gly Ala Val Ser Thr Leu Leu Gln Ala Pro Glu Leu
        515             520             525

Pro Thr Lys Thr Arg Thr Ser Arg Arg Ala Ala Glu His Arg Asp Thr
    530             535             540

Thr Asp Val Pro Glu Met Pro His Ser Lys Gly Gln Gly Glu Ser Asp
545             550             555             560

Pro Leu Asp His Glu Pro Ala Val Ser Pro Leu Leu Pro Arg Lys Glu
            565             570             575

Arg Gly Pro Pro Glu Gly Gly Leu Asn Glu Asp Glu Arg Leu Leu Pro
        580             585             590

Lys Asp Lys Lys Thr Asn Leu Phe Ser Ala Leu Ile Lys Lys Lys Lys
    595             600             605

Lys Thr Ala Pro Thr Pro Pro Lys Arg Ser Ser Ser Phe Arg Glu Met
    610             615             620

Asp Gly Gln Pro Glu Arg Arg Gly Ala Gly Glu Glu Glu Gly Arg Asp
625             630             635             640

Ile Ser Asn Gly Ala Leu Ala Phe Thr Pro Leu Asp Thr Ala Asp Pro
            645             650             655

Ala Lys Ser Pro Lys Pro Ser Asn Gly Ala Gly Val Pro Asn Gly Ala
            660             665             670

Leu Arg Glu Ser Gly Gly Ser Gly Phe Arg Ser Pro His Leu Trp Lys
    675             680             685

Lys Ser Ser Thr Leu Thr Ser Ser Arg Leu Ala Thr Gly Glu Glu Glu
    690             695             700

Gly Gly Gly Ser Ser Ser Lys Arg Phe Leu Arg Ser Cys Ser Ala Ser
705             710             715             720

```
Cys Val Pro His Gly Ala Lys Asp Thr Glu Trp Arg Ser Val Thr Leu
            725             730             735

Pro Arg Asp Leu Gln Ser Thr Gly Arg Gln Phe Asp Ser Ser Thr Phe
            740             745             750

Gly Gly His Lys Ser Glu Lys Pro Ala Leu Pro Arg Lys Arg Ala Gly
            755             760             765

Glu Asn Arg Ser Asp Gln Val Thr Arg Gly Thr Val Thr Pro Pro Pro
    770             775             780

Arg Leu Val Lys Lys Asn Glu Glu Ala Ala Asp Glu Val Phe Lys Asp
785             790             795             800

Ile Met Glu Ser Ser Pro Gly Ser Ser Pro Pro Asn Leu Thr Pro Lys
            805             810             815

Pro Leu Arg Arg Gln Val Thr Val Ala Pro Ala Ser Gly Leu Pro His
            820             825             830

Lys Glu Glu Ala Gly Lys Gly Ser Ala Leu Gly Thr Pro Ala Ala Ala
            835             840             845

Glu Pro Val Thr Pro Thr Ser Lys Ala Gly Ser Gly Ala Pro Gly Gly
    850             855             860

Thr Ser Lys Gly Pro Ala Glu Glu Ser Arg Val Arg Arg His Lys His
865             870             875             880

Ser Ser Glu Ser Pro Gly Arg Asp Lys Gly Lys Leu Ser Arg Leu Lys
            885             890             895

Pro Ala Pro Pro Pro Pro Ala Ala Ser Ala Gly Lys Ala Gly Gly
            900             905             910

Lys Pro Ser Gln Ser Pro Ser Gln Glu Ala Ala Gly Glu Ala Val Leu
            915             920             925

Gly Ala Lys Thr Lys Ala Thr Ser Leu Val Asp Ala Val Asn Ser Asp
    930             935             940

Ala Ala Lys Pro Ser Gln Pro Gly Glu Gly Leu Lys Lys Pro Val Leu
945             950             955             960

Pro Ala Thr Pro Lys Pro Gln Ser Ala Lys Pro Ser Gly Thr Pro Ile
            965             970             975

Ser Pro Ala Pro Val Pro Ser Thr Leu Pro Ser Ala Ser Ser Ala Leu
            980             985             990
```

```
Ala Gly Asp Gln Pro Ser Ser Thr  Ala Phe Ile Pro Leu  Ile Ser Thr
        995                 1000                 1005

Arg Val  Ser Leu Arg Lys Thr  Arg Gln Pro Pro Glu  Arg Ile Ala
    1010                 1015                 1020

Ser Gly  Ala Ile Thr Lys Gly  Val Val Leu Asp Ser  Thr Glu Ala
    1025                 1030                 1035

Leu Cys  Leu Ala Ile Ser Arg  Asn Ser Glu Gln Met  Ala Ser His
    1040                 1045                 1050

Ser Ala  Val Leu Glu Ala Gly  Lys Asn Leu Tyr Thr  Phe Cys Val
    1055                 1060                 1065

Ser Tyr  Val Asp Ser Ile Gln  Gln Met Arg Asn Lys  Phe Ala Phe
    1070                 1075                 1080

Arg Glu  Ala Ile Asn Lys Leu  Glu Asn Asn Leu Arg  Glu Leu Gln
    1085                 1090                 1095

Ile Cys  Pro Ala Thr Ala Gly  Ser Gly Pro Ala Ala  Thr Gln Asp
    1100                 1105                 1110

Phe Ser  Lys Leu Leu Ser Ser  Val Lys Glu Ile Ser  Asp Ile Val
    1115                 1120                 1125

Gln Arg
    1130


<210>  5
<211>  1146
<212>  PRT
<213>  Homo sapiens

<400>  5

Met Val Leu Gly Thr Val Leu Leu Pro Pro Asn Ser Tyr Gly Arg Asp
1               5                 10                 15

Gln Asp Thr Ser Leu Cys Cys Leu Cys Thr Glu Ala Ser Glu Ser Ala
            20                 25                 30

Leu Pro Asp Leu Thr Glu Ala Leu His Arg Pro Tyr Gly Cys Asp Val
            35                 40                 45

Glu Pro Gln Ala Leu Asn Glu Ala Ile Arg Trp Ser Ser Lys Glu Asn
        50                 55                 60

Leu Leu Gly Ala Thr Glu Ser Asp Pro Asn Leu Phe Val Ala Leu Tyr
65                 70                 75                 80
```

```
Asp Phe Val Ala Ser Gly Asp Asn Thr Leu Ser Ile Thr Lys Gly Glu
            85              90              95

Lys Leu Arg Val Leu Gly Tyr Asn Gln Asn Gly Glu Trp Ser Glu Val
            100             105             110

Arg Ser Lys Asn Gly Gln Gly Trp Val Pro Ser Asn Tyr Ile Thr Pro
            115             120             125

Val Asn Ser Leu Glu Lys His Ser Trp Tyr His Gly Pro Val Ser Arg
    130             135             140

Ser Ala Ala Glu Tyr Leu Leu Ser Ser Leu Ile Asn Gly Ser Phe Leu
145             150             155             160

Val Arg Glu Ser Glu Ser Ser Pro Gly Gln Leu Ser Ile Ser Leu Arg
            165             170             175

Tyr Glu Gly Arg Val Tyr His Tyr Arg Ile Asn Thr Thr Ala Asp Gly
            180             185             190

Lys Val Tyr Val Thr Ala Glu Ser Arg Phe Ser Thr Leu Ala Glu Leu
        195             200             205

Val His His His Ser Thr Val Ala Asp Gly Leu Val Thr Thr Leu His
    210             215             220

Tyr Pro Ala Pro Lys Cys Asn Lys Pro Thr Val Tyr Gly Val Ser Pro
225             230             235             240

Ile His Asp Lys Trp Glu Met Glu Arg Thr Asp Ile Thr Met Lys His
            245             250             255

Lys Leu Gly Gly Gly Gln Tyr Gly Glu Val Tyr Val Gly Val Trp Lys
            260             265             270

Lys Tyr Ser Leu Thr Val Ala Val Lys Thr Leu Lys Glu Asp Thr Met
        275             280             285

Glu Val Glu Glu Phe Leu Lys Glu Ala Ala Val Met Lys Glu Ile Lys
    290             295             300

His Pro Asn Leu Val Gln Leu Leu Gly Val Cys Thr Leu Glu Pro Pro
305             310             315             320

Phe Tyr Ile Val Thr Glu Tyr Met Pro Tyr Gly Asn Leu Leu Asp Tyr
            325             330             335

Leu Arg Glu Cys Asn Arg Glu Glu Val Thr Ala Val Val Leu Leu Tyr
            340             345             350
```

Met Ala Thr Gln Ile Ser Ser Ala Met Glu Tyr Leu Glu Lys Lys Asn
        355                 360                 365

Phe Ile His Arg Asp Leu Ala Ala Arg Asn Cys Leu Val Gly Glu Asn
    370                 375                 380

His Val Val Lys Val Ala Asp Phe Gly Leu Ser Arg Leu Met Thr Gly
385                 390                 395                 400

Asp Thr Tyr Thr Ala His Ala Gly Ala Lys Phe Pro Ile Lys Trp Thr
            405                 410                 415

Ala Pro Glu Ser Leu Ala Tyr Asn Thr Phe Ser Ile Lys Ser Asp Val
        420                 425                 430

Trp Ala Phe Gly Val Leu Leu Trp Glu Ile Ala Thr Tyr Gly Met Ser
        435                 440                 445

Pro Tyr Pro Gly Ile Asp Leu Ser Gln Val Tyr Asp Leu Leu Glu Lys
    450                 455                 460

Gly Tyr Arg Met Glu Gln Pro Glu Gly Cys Pro Pro Lys Val Tyr Glu
465                 470                 475                 480

Leu Met Arg Ala Cys Trp Lys Trp Ser Pro Ala Asp Arg Pro Ser Phe
            485                 490                 495

Ala Glu Thr His Gln Ala Phe Glu Thr Met Phe His Asp Ser Ser Ile
        500                 505                 510

Ser Glu Glu Val Ala Glu Glu Leu Gly Arg Ala Ala Ser Ser Ser Ser
        515                 520                 525

Val Val Pro Tyr Leu Pro Arg Leu Pro Ile Leu Pro Ser Lys Thr Arg
    530                 535                 540

Thr Leu Lys Lys Gln Val Glu Asn Lys Glu Asn Ile Glu Gly Ala Gln
545                 550                 555                 560

Asp Ala Thr Glu Asn Ser Ala Ser Ser Leu Ala Pro Gly Phe Ile Arg
            565                 570                 575

Gly Ala Gln Ala Ser Ser Gly Ser Pro Ala Leu Pro Arg Lys Gln Arg
        580                 585                 590

Asp Lys Ser Pro Ser Ser Leu Leu Glu Asp Ala Lys Glu Thr Cys Phe
        595                 600                 605

Thr Arg Asp Arg Lys Gly Gly Phe Phe Ser Ser Phe Met Lys Lys Arg
    610                 615                 620

31

```
Asn Ala Pro Thr Pro Pro Lys Arg Ser Ser Ser Phe Arg Glu Met Glu
625             630             635             640

Asn Gln Pro His Lys Lys Tyr Glu Leu Thr Gly Asn Phe Ser Ser Val
            645             650             655

Ala Ser Leu Gln His Ala Asp Gly Phe Ser Phe Thr Pro Ala Gln Gln
            660             665             670

Glu Ala Asn Leu Val Pro Pro Lys Cys Tyr Gly Gly Ser Phe Ala Gln
            675             680             685

Arg Asn Leu Cys Asn Asp Asp Gly Gly Gly Gly Gly Ser Gly Thr
    690             695             700

Ala Gly Gly Gly Trp Ser Gly Ile Thr Gly Phe Phe Thr Pro Arg Leu
705             710             715             720

Ile Lys Lys Thr Leu Gly Leu Arg Ala Gly Lys Pro Thr Ala Ser Asp
            725             730             735

Asp Thr Ser Lys Pro Phe Pro Arg Ser Asn Ser Thr Ser Ser Met Ser
            740             745             750

Ser Gly Leu Pro Glu Gln Asp Arg Met Ala Met Thr Leu Pro Arg Asn
            755             760             765

Cys Gln Arg Ser Lys Leu Gln Leu Glu Arg Thr Val Ser Thr Ser Ser
    770             775             780

Gln Pro Glu Glu Asn Val Asp Arg Ala Asn Asp Met Leu Pro Lys Lys
785             790             795             800

Ser Glu Glu Ser Ala Ala Pro Ser Arg Glu Arg Pro Lys Ala Lys Leu
            805             810             815

Leu Pro Arg Gly Ala Thr Ala Leu Pro Leu Arg Thr Pro Ser Gly Asp
            820             825             830

Leu Ala Ile Thr Glu Lys Asp Pro Pro Gly Val Gly Val Ala Gly Val
            835             840             845

Ala Ala Ala Pro Lys Gly Lys Glu Lys Asn Gly Gly Ala Arg Leu Gly
    850             855             860

Met Ala Gly Val Pro Glu Asp Gly Glu Gln Pro Gly Trp Pro Ser Pro
865             870             875             880

Ala Lys Ala Ala Pro Val Leu Pro Thr Thr His Asn His Lys Val Pro
            885             890             895
```

32

```
Val Leu Ile Ser Pro Thr Leu Lys His Thr Pro Ala Asp Val Gln Leu
             900             905             910

Ile Gly Thr Asp Ser Gln Gly Asn Lys Phe Lys Leu Leu Ser Glu His
             915             920             925

Gln Val Thr Ser Ser Gly Asp Lys Asp Arg Pro Arg Arg Val Lys Pro
    930             935             940

Lys Cys Ala Pro Pro Pro Pro Val Met Arg Leu Leu Gln His Pro
945             950             955             960

Ser Ile Cys Ser Asp Pro Thr Glu Glu Pro Thr Ala Leu Thr Ala Gly
             965             970             975

Gln Ser Thr Ser Glu Thr Gln Glu Gly Gly Lys Lys Ala Ala Leu Gly
             980             985             990

Ala Val Pro Ile Ser Gly Lys Ala Gly Arg Pro Val Met Pro Pro Pro
             995             1000            1005

Gln Val Pro Leu Pro Thr Ser Ser Ile Ser Pro Ala Lys Met Ala
    1010            1015            1020

Asn Gly Thr Ala Gly Thr Lys Val Ala Leu Arg Lys Thr Lys Gln
    1025            1030            1035

Ala Ala Glu Lys Ile Ser Ala Asp Lys Ile Ser Lys Glu Ala Leu
    1040            1045            1050

Leu Glu Cys Ala Asp Leu Leu Ser Ser Ala Leu Thr Glu Pro Val
    1055            1060            1065

Pro Asn Ser Gln Leu Val Asp Thr Gly His Gln Leu Leu Asp Tyr
    1070            1075            1080

Cys Ser Gly Tyr Val Asp Cys Ile Pro Gln Thr Arg Asn Lys Phe
    1085            1090            1095

Ala Phe Arg Glu Ala Val Ser Lys Leu Glu Leu Ser Leu Gln Glu
    1100            1105            1110

Leu Gln Val Ser Ser Ala Ala Ala Gly Val Pro Gly Thr Asn Pro
    1115            1120            1125

Val Leu Asn Asn Leu Leu Ser Cys Val Gln Glu Ile Ser Asp Val
    1130            1135            1140

Val Gln Arg
    1145
```

33

EP 2 505 572 A1

<210> 6
<211> 498
<212> PRT
<213> Homo sapiens

<400> 6

Met Asp Tyr Asp Phe Lys Ala Lys Leu Ala Ala Glu Arg Glu Arg Val
1               5                   10                  15

Glu Asp Leu Phe Glu Tyr Glu Gly Cys Lys Val Gly Arg Gly Thr Tyr
                20                  25                  30

Gly His Val Tyr Lys Ala Arg Arg Lys Asp Gly Lys Asp Glu Lys Glu
            35                  40                  45

Tyr Ala Leu Lys Gln Ile Glu Gly Thr Gly Ile Ser Met Ser Ala Cys
        50                  55                  60

Arg Glu Ile Ala Leu Leu Arg Glu Leu Lys His Pro Asn Val Ile Ala
65                  70                  75                  80

Leu Gln Lys Val Phe Leu Ser His Ser Asp Arg Lys Val Trp Leu Leu
                85                  90                  95

Phe Asp Tyr Ala Glu His Asp Leu Trp His Ile Ile Lys Phe His Arg
            100                 105                 110

Ala Ser Lys Ala Asn Lys Lys Pro Met Gln Leu Pro Arg Ser Met Val
        115                 120                 125

Lys Ser Leu Leu Tyr Gln Ile Leu Asp Gly Ile His Tyr Leu His Ala
    130                 135                 140

Asn Trp Val Leu His Arg Asp Leu Lys Pro Ala Asn Ile Leu Val Met
145                 150                 155                 160

Gly Glu Gly Pro Glu Arg Gly Arg Val Lys Ile Ala Asp Met Gly Phe
                165                 170                 175

Ala Arg Leu Phe Asn Ser Pro Leu Lys Pro Leu Ala Asp Leu Asp Pro
            180                 185                 190

Val Val Val Thr Phe Trp Tyr Arg Ala Pro Glu Leu Leu Leu Gly Ala
        195                 200                 205

Arg His Tyr Thr Lys Ala Ile Asp Ile Trp Ala Ile Gly Cys Ile Phe
    210                 215                 220

Ala Glu Leu Leu Thr Ser Glu Pro Ile Phe His Cys Arg Gln Glu Asp
225                 230                 235                 240

Ile Lys Thr Ser Asn Pro Phe His His Asp Gln Leu Asp Arg Ile Phe

34

                    245                    250                    255

Ser Val Met Gly Phe Pro Ala Asp Lys Asp Trp Glu Asp Ile Arg Lys
            260                265                270

Met Pro Glu Tyr Pro Thr Leu Gln Lys Asp Phe Arg Arg Thr Thr Tyr
        275                280                285

Ala Asn Ser Ser Leu Ile Lys Tyr Met Glu Lys His Lys Val Lys Pro
    290                295                300

Asp Ser Lys Val Phe Leu Leu Leu Gln Lys Leu Leu Thr Met Asp Pro
305                310                315                320

Thr Lys Arg Ile Thr Ser Glu Gln Ala Leu Gln Asp Pro Tyr Phe Gln
                325                330                335

Glu Asp Pro Leu Pro Thr Leu Asp Val Phe Ala Gly Cys Gln Ile Pro
            340                345                350

Tyr Pro Lys Arg Glu Phe Leu Asn Glu Asp Asp Pro Glu Glu Lys Gly
        355                360                365

Asp Lys Asn Gln Gln Gln Gln Gln Asn Gln His Gln Gln Pro Thr Ala
    370                375                380

Pro Pro Gln Gln Ala Ala Ala Pro Pro Gln Ala Pro Pro Pro Gln Gln
385                390                395                400

Asn Ser Thr Gln Thr Asn Gly Thr Ala Gly Gly Ala Gly Ala Gly Val
            405                410                415

Gly Gly Thr Gly Ala Gly Leu Gln His Ser Gln Asp Ser Ser Leu Asn
            420                425                430

Gln Val Pro Pro Asn Lys Lys Pro Arg Leu Gly Pro Ser Gly Ala Asn
        435                440                445

Ser Gly Gly Pro Val Met Pro Ser Asp Tyr Gln His Ser Ser Ser Arg
    450                455                460

Leu Asn Tyr Gln Ser Ser Val Gln Gly Ser Ser Gln Ser Gln Ser Thr
465                470                475                480

Leu Gly Tyr Ser Ser Ser Ser Gln Gln Ser Ser Gln Tyr His Pro Ser
            485                490                495

His Gln

<210> 7

```
<211>   298
<212>   PRT
<213>   Homo sapiens

<400>   7

Met Glu Asn Phe Gln Lys Val Glu Lys Ile Gly Glu Gly Thr Tyr Gly
1               5                  10                 15

Val Val Tyr Lys Ala Arg Asn Lys Leu Thr Gly Glu Val Val Ala Leu
            20                 25                 30

Lys Lys Ile Arg Leu Asp Thr Glu Thr Glu Gly Val Pro Ser Thr Ala
            35                 40                 45

Ile Arg Glu Ile Ser Leu Leu Lys Glu Leu Asn His Pro Asn Ile Val
        50                 55                 60

Lys Leu Leu Asp Val Ile His Thr Glu Asn Lys Leu Tyr Leu Val Phe
65                 70                 75                 80

Glu Phe Leu His Gln Asp Leu Lys Lys Phe Met Asp Ala Ser Ala Leu
                85                 90                 95

Thr Gly Ile Pro Leu Pro Leu Ile Lys Ser Tyr Leu Phe Gln Leu Leu
            100                105                110

Gln Gly Leu Ala Phe Cys His Ser His Arg Val Leu His Arg Asp Leu
        115                120                125

Lys Pro Gln Asn Leu Leu Ile Asn Thr Glu Gly Ala Ile Lys Leu Ala
    130                135                140

Asp Phe Gly Leu Ala Arg Ala Phe Gly Val Pro Val Arg Thr Tyr Thr
145                150                155                160

His Glu Val Val Thr Leu Trp Tyr Arg Ala Pro Glu Ile Leu Leu Gly
                165                170                175

Cys Lys Tyr Tyr Ser Thr Ala Val Asp Ile Trp Ser Leu Gly Cys Ile
            180                185                190

Phe Ala Glu Met Val Thr Arg Arg Ala Leu Phe Pro Gly Asp Ser Glu
        195                200                205

Ile Asp Gln Leu Phe Arg Ile Phe Arg Thr Leu Gly Thr Pro Asp Glu
    210                215                220

Val Val Trp Pro Gly Val Thr Ser Met Pro Asp Tyr Lys Pro Ser Phe
225                230                235                240

Pro Lys Trp Ala Arg Gln Asp Phe Ser Lys Val Val Pro Pro Leu Asp
            245                250                255
```

Glu Asp Gly Arg Ser Leu Leu Ser Gln Met Leu His Tyr Asp Pro Asn
              260             265             270

Lys Arg Ile Ser Ala Lys Ala Ala Leu Ala His Pro Phe Phe Gln Asp
          275             280             285

Val Thr Lys Pro Val Pro His Leu Arg Leu
    290             295

<210> 8
<211> 305
<212> PRT
<213> Homo sapiens

<400> 8

Met Asp Met Phe Gln Lys Val Glu Lys Ile Gly Glu Gly Thr Tyr Gly
1               5               10              15

Val Val Tyr Lys Ala Lys Asn Arg Glu Thr Gly Gln Leu Val Ala Leu
            20              25              30

Lys Lys Ile Arg Leu Asp Leu Glu Met Glu Gly Val Pro Ser Thr Ala
        35              40              45

Ile Arg Glu Ile Ser Leu Leu Lys Glu Leu Lys His Pro Asn Ile Val
    50              55              60

Arg Leu Leu Asp Val Val His Asn Glu Arg Lys Leu Tyr Leu Val Phe
65              70              75              80

Glu Phe Leu Ser Gln Asp Leu Lys Lys Tyr Met Asp Ser Thr Pro Gly
            85              90              95

Ser Glu Leu Pro Leu His Leu Ile Lys Ser Tyr Leu Phe Gln Leu Leu
            100             105             110

Gln Gly Val Ser Phe Cys His Ser His Arg Val Ile His Arg Asp Leu
        115             120             125

Lys Pro Gln Asn Leu Leu Ile Asn Glu Leu Gly Ala Ile Lys Leu Ala
    130             135             140

Asp Phe Gly Leu Ala Arg Ala Phe Gly Val Pro Leu Arg Thr Tyr Thr
145             150             155             160

His Glu Val Val Thr Leu Trp Tyr Arg Ala Pro Glu Ile Leu Leu Gly
            165             170             175

Ser Lys Phe Tyr Thr Thr Ala Val Asp Ile Trp Ser Ile Gly Cys Ile
            180             185             190

```
Phe Ala Glu Met Val Thr Arg Lys Ala Leu Phe Pro Gly Asp Ser Glu
        195                 200             205

Ile Asp Gln Leu Phe Arg Ile Phe Arg Met Leu Gly Thr Pro Ser Glu
        210             215             220

Asp Thr Trp Pro Gly Val Thr Gln Leu Pro Asp Tyr Lys Gly Ser Phe
225                 230             235                 240

Pro Lys Trp Thr Arg Lys Gly Leu Glu Glu Ile Val Pro Asn Leu Glu
            245             250                 255

Pro Glu Gly Arg Asp Leu Leu Met Gln Leu Leu Gln Tyr Asp Pro Ser
            260             265             270

Gln Arg Ile Thr Ala Lys Thr Ala Leu Ala His Pro Tyr Phe Ser Ser
            275             280             285

Pro Glu Pro Ser Pro Ala Ala Arg Gln Tyr Val Leu Gln Arg Phe Arg
        290             295             300

His
305


<210>  9
<211>  292
<212>  PRT
<213>  Homo sapiens

<400>  9

Met Gln Lys Tyr Glu Lys Leu Glu Lys Ile Gly Glu Gly Thr Tyr Gly
1               5                   10              15

Thr Val Phe Lys Ala Lys Asn Arg Glu Thr His Glu Ile Val Ala Leu
            20              25              30

Lys Arg Val Arg Leu Asp Asp Asp Asp Glu Gly Val Pro Ser Ser Ala
            35              40              45

Leu Arg Glu Ile Cys Leu Leu Lys Glu Leu Lys His Lys Asn Ile Val
        50              55              60

Arg Leu His Asp Val Leu His Ser Asp Lys Lys Leu Thr Leu Val Phe
65                  70              75                  80

Glu Phe Cys Asp Gln Asp Leu Lys Lys Tyr Phe Asp Ser Cys Asn Gly
                85              90              95

Asp Leu Asp Pro Glu Ile Val Lys Ser Phe Leu Phe Gln Leu Leu Lys
            100             105             110
```

```
Gly Leu Gly Phe Cys His Ser Arg Asn Val Leu His Arg Asp Leu Lys
        115             120             125

Pro Gln Asn Leu Leu Ile Asn Arg Asn Gly Glu Leu Lys Leu Ala Asp
        130             135             140

Phe Gly Leu Ala Arg Ala Phe Gly Ile Pro Val Arg Cys Tyr Ser Ala
145             150             155             160

Glu Val Val Thr Leu Trp Tyr Arg Pro Pro Asp Val Leu Phe Gly Ala
                165             170             175

Lys Leu Tyr Ser Thr Ser Ile Asp Met Trp Ser Ala Gly Cys Ile Phe
            180             185             190

Ala Glu Leu Ala Asn Ala Gly Arg Pro Leu Phe Pro Gly Asn Asp Val
            195             200             205

Asp Asp Gln Leu Lys Arg Ile Phe Arg Leu Leu Gly Thr Pro Thr Glu
    210             215             220

Glu Gln Trp Pro Ser Met Thr Lys Leu Pro Asp Tyr Lys Pro Tyr Pro
225             230             235             240

Met Tyr Pro Ala Thr Thr Ser Leu Val Asn Val Val Pro Lys Leu Asn
            245             250             255

Ala Thr Gly Arg Asp Leu Leu Gln Asn Leu Leu Lys Cys Asn Pro Val
            260             265             270

Gln Arg Ile Ser Ala Glu Glu Ala Leu Gln His Pro Tyr Phe Ser Asp
        275             280             285

Phe Cys Pro Pro
    290
```

<210> 10
<211> 346
<212> PRT
<213> Homo sapiens

<400> 10

```
Met Ala Leu Asp Val Lys Ser Arg Ala Lys Arg Tyr Glu Lys Leu Asp
1               5               10              15

Phe Leu Gly Glu Gly Gln Phe Ala Thr Val Tyr Lys Ala Arg Asp Lys
            20              25              30

Asn Thr Asn Gln Ile Val Ala Ile Lys Lys Ile Lys Leu Gly His Arg
        35              40              45

Ser Glu Ala Lys Asp Gly Ile Asn Arg Thr Ala Leu Arg Glu Ile Lys
```

39

|  | | 50 | | | | 55 | | | | 60 | |
|---|---|---|---|---|---|---|---|---|---|---|---|

Leu Leu Gln Glu Leu Ser His Pro Asn Ile Ile Gly Leu Leu Asp Ala
65                    70                    75                    80

Phe Gly His Lys Ser Asn Ile Ser Leu Val Phe Asp Phe Met Glu Thr
                    85                    90                    95

Asp Leu Glu Val Ile Ile Lys Asp Asn Ser Leu Val Leu Thr Pro Ser
                    100                   105                   110

His Ile Lys Ala Tyr Met Leu Met Thr Leu Gln Gly Leu Glu Tyr Leu
                    115                   120                   125

His Gln His Trp Ile Leu His Arg Asp Leu Lys Pro Asn Asn Leu Leu
                    130                   135                   140

Leu Asp Glu Asn Gly Val Leu Lys Leu Ala Asp Phe Gly Leu Ala Lys
145                   150                   155                   160

Ser Phe Gly Ser Pro Asn Arg Ala Tyr Thr His Gln Val Val Thr Arg
                    165                   170                   175

Trp Tyr Arg Ala Pro Glu Leu Leu Phe Gly Ala Arg Met Tyr Gly Val
                    180                   185                   190

Gly Val Asp Met Trp Ala Val Gly Cys Ile Leu Ala Glu Leu Leu Leu
                    195                   200                   205

Arg Val Pro Phe Leu Pro Gly Asp Ser Asp Leu Asp Gln Leu Thr Arg
                    210                   215                   220

Ile Phe Glu Thr Leu Gly Thr Pro Thr Glu Glu Gln Trp Pro Asp Met
225                   230                   235                   240

Cys Ser Leu Pro Asp Tyr Val Thr Phe Lys Ser Phe Pro Gly Ile Pro
                    245                   250                   255

Leu His His Ile Phe Ser Ala Ala Gly Asp Asp Leu Leu Asp Leu Ile
                    260                   265                   270

Gln Gly Leu Phe Leu Phe Asn Pro Cys Ala Arg Ile Thr Ala Thr Gln
                    275                   280                   285

Ala Leu Lys Met Lys Tyr Phe Ser Asn Arg Pro Gly Pro Thr Pro Gly
                    290                   295                   300

Cys Gln Leu Pro Arg Pro Asn Cys Pro Val Glu Thr Leu Lys Glu Gln
305                   310                   315                   320

Ser Asn Pro Ala Leu Ala Ile Lys Arg Lys Arg Thr Glu Ala Leu Glu

325                330             335

```
Gln Gly Gly Leu Pro Lys Lys Leu Ile Phe
            340                 345


<210>   11
<211>   312
<212>   PRT
<213>   Homo sapiens

<400>   11

Gly Cys Lys Val Gly Arg Gly Thr Tyr Gly His Val Tyr Lys Ala Lys
1               5                   10                  15

Arg Lys Asp Gly Lys Asp Asp Lys Asp Tyr Ala Leu Lys Gln Ile Glu
            20                  25                  30

Gly Thr Gly Ile Ser Met Ser Ala Cys Arg Glu Ile Ala Leu Leu Arg
            35              40                  45

Glu Leu Lys His Pro Asn Val Ile Ser Leu Gln Lys Val Phe Leu Ser
        50              55                  60

His Ala Asp Arg Lys Val Trp Leu Leu Phe Asp Tyr Ala Glu His Asp
65              70                  75                  80

Leu Trp His Ile Ile Lys Phe His Arg Ala Ser Lys Ala Asn Lys Lys
                85                  90                  95

Pro Val Gln Leu Pro Arg Gly Met Val Lys Ser Leu Leu Tyr Gln Ile
            100                 105                 110

Leu Asp Gly Ile His Tyr Leu His Ala Asn Trp Val Leu His Arg Asp
            115                 120                 125

Leu Lys Pro Ala Asn Ile Leu Val Met Gly Glu Gly Pro Glu Arg Gly
        130                 135                 140

Arg Val Lys Ile Ala Asp Met Gly Phe Ala Arg Leu Phe Asn Ser Pro
145                 150                 155                 160

Leu Lys Pro Leu Ala Asp Leu Asp Pro Val Val Val Thr Phe Trp Tyr
            165                 170                 175

Arg Ala Pro Glu Leu Leu Leu Gly Ala Arg His Tyr Thr Lys Ala Ile
            180                 185                 190

Asp Ile Trp Ala Ile Gly Cys Ile Phe Ala Glu Leu Leu Thr Ser Glu
            195                 200                 205

Pro Ile Phe His Cys Arg Gln Glu Asp Ile Lys Thr Ser Asn Pro Tyr
            210                 215                 220
```

```
His His Asp Gln Leu Asp Arg Ile Phe Asn Val Met Gly Phe Pro Ala
225             230             235             240

Asp Lys Asp Trp Glu Asp Ile Lys Lys Met Pro Glu His Ser Thr Leu
                245             250             255

Met Lys Asp Phe Arg Arg Asn Thr Tyr Thr Asn Cys Ser Leu Ile Lys
                260             265             270

Tyr Met Glu Lys His Lys Val Lys Pro Asp Ser Lys Ala Phe His Leu
        275             280             285

Leu Gln Lys Leu Leu Thr Met Asp Pro Ile Lys Arg Ile Thr Ser Glu
        290             295             300

Gln Ala Met Gln Asp Pro Tyr Phe
305             310
```

```
<210>   12
<211>   372
<212>   PRT
<213>   Homo sapiens

<400>   12
```

```
Met Ala Lys Gln Tyr Asp Ser Val Glu Cys Pro Phe Cys Asp Glu Val
1               5               10              15

Ser Lys Tyr Glu Lys Leu Ala Lys Ile Gly Gln Gly Thr Phe Gly Glu
            20              25              30

Val Phe Lys Ala Arg His Arg Lys Thr Gly Gln Lys Val Ala Leu Lys
        35              40              45

Lys Val Leu Met Glu Asn Glu Lys Glu Gly Phe Pro Ile Thr Ala Leu
        50              55              60

Arg Glu Ile Lys Ile Leu Gln Leu Leu Lys His Glu Asn Val Val Asn
65              70              75              80

Leu Ile Glu Ile Cys Arg Thr Lys Ala Ser Pro Tyr Asn Arg Cys Lys
                85              90              95

Gly Ser Ile Tyr Leu Val Phe Asp Phe Cys Glu His Asp Leu Ala Gly
            100             105             110

Leu Leu Ser Asn Val Leu Val Lys Phe Thr Leu Ser Glu Ile Lys Arg
        115             120             125

Val Met Gln Met Leu Leu Asn Gly Leu Tyr Tyr Ile His Arg Asn Lys
        130             135             140
```

```
Ile Leu His Arg Asp Met Lys Ala Ala Asn Val Leu Ile Thr Arg Asp
145             150             155             160

Gly Val Leu Lys Leu Ala Asp Phe Gly Leu Ala Arg Ala Phe Ser Leu
            165             170             175

Ala Lys Asn Ser Gln Pro Asn Arg Tyr Thr Asn Arg Val Val Thr Leu
            180             185             190

Trp Tyr Arg Pro Pro Glu Leu Leu Leu Gly Glu Arg Asp Tyr Gly Pro
        195             200             205

Pro Ile Asp Leu Trp Gly Ala Gly Cys Ile Met Ala Glu Met Trp Thr
    210             215             220

Arg Ser Pro Ile Met Gln Gly Asn Thr Glu Gln His Gln Leu Ala Leu
225             230             235             240

Ile Ser Gln Leu Cys Gly Ser Ile Thr Pro Glu Val Trp Pro Asn Val
            245             250             255

Asp Asn Tyr Glu Leu Tyr Glu Lys Leu Glu Leu Val Lys Gly Gln Lys
            260             265             270

Arg Lys Val Lys Asp Arg Leu Lys Ala Tyr Val Arg Asp Pro Tyr Ala
            275             280             285

Leu Asp Leu Ile Asp Lys Leu Leu Val Leu Asp Pro Ala Gln Arg Ile
    290             295             300

Asp Ser Asp Asp Ala Leu Asn His Asp Phe Phe Trp Ser Asp Pro Met
305             310             315             320

Pro Ser Asp Leu Lys Gly Met Leu Ser Thr His Leu Thr Ser Met Phe
            325             330             335

Glu Tyr Leu Ala Pro Pro Arg Arg Lys Gly Ser Gln Ile Thr Gln Gln
            340             345             350

Ser Thr Asn Gln Ser Arg Asn Pro Ala Thr Thr Asn Gln Thr Glu Phe
            355             360             365

Glu Arg Val Phe
    370
```

# EP 2 505 572 A1

**Description**

The present invention relates to a kinase containing exactly one label in the C helix, (a) wherein said label is a fluorophore, a spin label or an isotope label, (a)(i) wherein said fluorophore and said spin label are covalently bound to a thiol group or side chain amino group of an amino acid residue of said C helix, (a)(ii) wherein said isotope label is a moiety comprising one or more atoms which are isotopes with non-zero nuclear spin, said moiety being covalently bound to a thiol group or side chain amino group of an amino acid residue of said C helix, or said isotope label is an amino acid residue of said C helix, said amino acid residue comprising one or more atoms which are isotopes with non-zero nuclear spin, wherein said isotopes with non-zero nuclear spin are enriched as compared to the natural occurrence thereof.

In this specification, a number of documents including patent applications and manufacturer's manuals are cited. The disclosure of these documents, while not considered relevant for the patentability of this invention, is herewith incorporated by reference in its entirety. More specifically, all reference documents are incorporated by reference to the same extent as if each individual document was specifically and individually indicated to be incorporated by reference.

Protein kinases regulate numerous cellular processes. The improved understanding of aberrantly regulated kinase signaling in cancer biology (Gschwind and Fischer, 2004) has lead to the development of small organic molecules that are used to specifically target unwanted kinase activities and initiated the area of targeted cancer therapies (Zhang et al., 2009). Most kinase inhibitors are Type I inhibitors such as dasatinib (Sprycel®), bind to the active "DFG-in" conformation of the kinase and compete with ATP in order to form hydrogen bonds with the kinase hinge region. In this conformation, the regulatory activation loop containing the conserved DFG (Asp-Phe-Gly) motif is open and extended, which allows ATP and substrates to bind (Knighton et al., 1991). The adenine of ATP forms a hydrogen bond with the hinge region of the kinase - a short, flexible region located between the N- and C-terminal lobes of the kinase domain while the $\beta$ and $\gamma$ phosphates of ATP are coordinated by a network of ionic and hydrogen bonding interactions with several structural elements, including $Mg^{2+}$ or $Mn^{2+}$ ions, the Asp side chain of the conserved DFG motif, and amino acid residues in the glycine-rich loop located above the ATP binding cleft (Aimes et al., 2000). However, the development of these types of inhibitors is challenged by an increasingly exhausted chemical space within the ATP binding site, poor inhibitor selectivity and efficacy as well as the emergence of drug resistance.

Current medicinal chemistry research attempts to overcome these bottlenecks in the development of effective long-term therapies by identifying and developing inhibitors that target alternative (i.e. allosteric) binding sites and/or stabilize inactive kinase conformations which are enzymatically incompetent (Zhang et al., 2009; Adrian et al., 2006; Calleja et al., 2009; Fischmann et al., 2009; Kirkland and McInnes, 2009). One of these sites is only present in the inactive "DFG-out" kinase conformation and is moving to the forefront of kinase research. The DFG-out conformation results from structural changes in the activation loop induced by a flip of the highly-conserved DFG motif (Liu and Gray, 2006; Pargellis et al., 2002), an event which also exposes a less-conserved allosteric site adjacent to the ATP binding site. Type II and Type III inhibitors bind to this less conserved allosteric site and are believed to have superior selectivity profiles, improved pharmacological properties (Copeland et al., 2006) and offer new opportunities for drug development (Liu and Gray, 2006). More specifically, Type II inhibitors such as sorafenib (Nexavar®, Wan et al., 2004), imatinib (Gleevec®, Nagar et al., 2002) and BIRB-796, a selective inhibitor of p38$\alpha$ (Pargellis et al., 2002), bind to the hinge region and are ATP-competitive but extend into this allosteric site while Type III inhibitors bind exclusively within the allosteric pocket (Pargellis et al., 2002; Simard et al., A new screening assay for allosteric inhibitors of cSrc. Nat Chem Bio/. 2009 Jun;5(6):394-6; Simard et al., Development of a fluorescent-tagged kinase assay system for the detection and characterization of allosteric kinase inhibitors J Am Chem Soc. 2009 Sep 23;131(37):13286-96; Simard et al., High-throughput screening to identify inhibitors which stabilize inactive kinase conformations in p38alpha. J Am Chem Soc. 2009 Dec 30;131(51):18478-88; Simard et al., Fluorophore labeling of the glycine-rich loop as a method of identifying inhibitors that bind to active and inactive kinase conformations Am Chem Soc. 2010 Mar 31;132(12):4152-60). Until recently, approaches that allowed for the unambiguous identification of inhibitors which stabilize the inactive DFG-out conformation fell short or were not compatible with the high-throughput screening formats used by academia and industry to identify new hit compounds (Annis et al., 2004; Vogtherr et al., 2006), thus highlighting the need for innovative new approaches to detect and characterize such ligands.

The attachment of fluorophores to proteins is an approach used to detect conformational changes in protein structure in response to ligand binding. In addition to the commercially-available probe acrylodan-labeled fatty acid binding protein (ADIFAB; Molecular Probes), which measures the concentration of unbound fatty acids in buffer (Richieri et al., 1999), this approach has been applied to proteins such as acetylcholine binding protein (Hibbs et al., 2004), interleukin-1$\beta$ (Yem et al., 1992) and certain sugar and amino acid binding proteins (de Lorimier et al., 2002).

Besides conventional kinase assays for the screening of modulators of kinase activity, various approaches have recently been developed. However, many of these approaches suffer from major drawbacks. For example, Annis et al. (2004) describe an approach using affinity selection-mass spectrometry (AS-MS). This method is described as suitable for high-throughput screening. However, a size exclusion chromatography step has to be applied prior to the examination of each probe which is time-consuming and requires a lot of material.

De Lorimier et al. (2002) describe a family of biosensors based on bacterial proteins binding to small molecule ligands which were modified and labeled with different environmentally sensitive fluorophores. Upon ligand binding, the fluorophores alter their emission wavelength and/or intensity thus indicating the presence and/or concentration of the specific ligand bound to a probe. However, the labeling of kinases and the use of said kinases in the screening for specific inhibitors is neither disclosed nor suggested.

More recently, two additional binding assays based on the displacement of prebound probes from p38α kinase were also reported: one made use of a fluorophore-labeled inhibitor (Tecle et al., 2009) and the other employed an enzyme fragment complementation-based approach (Kluter et al., 2009). In the latter case, a chemiluminescence read-out was generated by the displacement of a prebound inhibitor-peptide probe, which then complements and activates β-galactosidase to catalyze a chemiluminescence reaction that serves as the assay read-out. Although these approaches were demonstrated to be suitable for determining the affinities of displacing ligands using end point measurements, analysis of kinetic parameters ($k_{on}$ and $k_{off}$) is less straightforward since signal detection is rate-limited by the well-characterized slow dissociation of the chosen pyrazolourea-based probes from p38α (Pargellis et al., 2002).

Accordingly, it would be desirable to have alternative or improved means and methods for screening for specific kinase inhibitors. The solution to this technical problem is achieved by providing the embodiments characterized in the claims.

Accordingly, the present invention provides a kinase containing exactly one label in the C helix, (a) wherein said label is a fluorophore, a spin label or an isotope label, (a)(i) wherein said fluorophore and said spin label are covalently bound to a thiol group or side chain amino group of an amino acid residue of said C helix, (a)(ii) wherein said isotope label is a moiety comprising one or more atoms which are isotopes with non-zero nuclear spin, said moiety being covalently bound to a thiol group or side chain amino group of an amino acid residue of said C helix, or said isotope label is an amino acid residue of said C helix, said amino acid residue comprising one or more atoms which are isotopes with non-zero nuclear spin, wherein said isotopes with non-zero nuclear spin are enriched as compared to the natural occurrence thereof.

The term "kinase" is well-known in the art and refers to a type of enzyme that transfers phosphate groups from high-energy donor molecules, such as ATP, to specific target molecules such as proteins. Kinases are classified under the enzyme commission (EC) number 2.7. Kinases which phosphorylate proteins are also referred to as protein kinases. Preferably, the kinases according to the invention are protein kinases. According to substrate specificity, protein kinases can be subdivided into serine/threonine kinases (EC 2.7.11, e.g. p38α), tyrosine kinases (EC 2.7.10, e.g. the EGFR kinase domain), histidine kinases (EC 2.7.13), aspartic acid/glutamic acid kinases and mixed kinases (EC 2.7.12) which have more than one specificity (e.g. MEK being specific for serine/threonine and tyrosine). All protein kinases comprise or consist of at least a protein kinase domain which comprises the structural elements discussed herein. Amino acid sequences, secondary and tertiary structure of protein kinases contain a plurality of conserved elements which are well-known in the art; see, for example, Hanks, Steven K., Hunter, Tony. The eukaryotic protein kinase superfamily: kinase (catalytic) domain structure and classification. Academic Press London 1995, 9, 576-596; Rabiller, M., Getlik, M., Klüter, S., Richters, A., Tückmantel, S., Simard, J.R., Rauh, D. Proteus in the world of proteins: Conformational changes in protein kinases. Arch. Pharm. Chem. Life Sci. 2010, 343, 193-206; and Serota Taylor, S., Radzio-Andzelm, Elzbieta. Three protein kinase structures define a common motif. Structure 1994, 2:345-355.

The term "label" is well-known in the art. In the present case, the label according to invention is to be selected from a fluorophore, a spin label and an isotope label which are discussed in more detail below. Importantly, the label according to the invention is a moiety which does not occur in the wild type form of said kinase. For example, a fluorophore as comprised in a naturally occurring amino acid is not a label according to the invention. Similarly, an amino acid containing a $^{13}$C nucleotide is not a label according to the invention if the frequency of $^{13}$C at the given position merely arises from the natural distribution of isotopes.

The term "wild type form" as used herein is meant to distinguish naturally occurring kinases from (i) kinases which have sequences that are modified in accordance with the present invention for the purpose of labeling and (ii) kinases containing a label in C helix according to the invention. Accordingly, the term "wild type form" includes naturally occurring mutants such as, for example, disease-relevant mutants, provided that those mutants are neither labeled in accordance with the invention nor have a sequence which is modified for the purpose of labeling. It is understood that in such naturally occurring mutant forms the mutated position is generally irrelevant for and not useful for labeling.

In other words, a given kinase may occur in at least three different forms. First, there is the wild type form of the kinase which, for the purpose of labeling, might require certain modifications of the sequence such as to introduction of a site amenable to labeling. Such modifications are further detailed below. Another type of modification which might be necessary is the removal of one or more sites where labeling is not desired. In either of the two cases, a difference in sequence will arise between the wild type form of the kinase and the kinase which is amenable to labeling. Upon labeling, which may be achieved by reacting with reactive forms of fluorophores, spin labels and isotope labels, a further form is obtained which is the kinase according to the invention. Said kinase according to the invention differs from the wild type form thereof in that it is labeled, and, if the sequence of the wild type form was not suitable for labeling, by one or more sequence modifications.

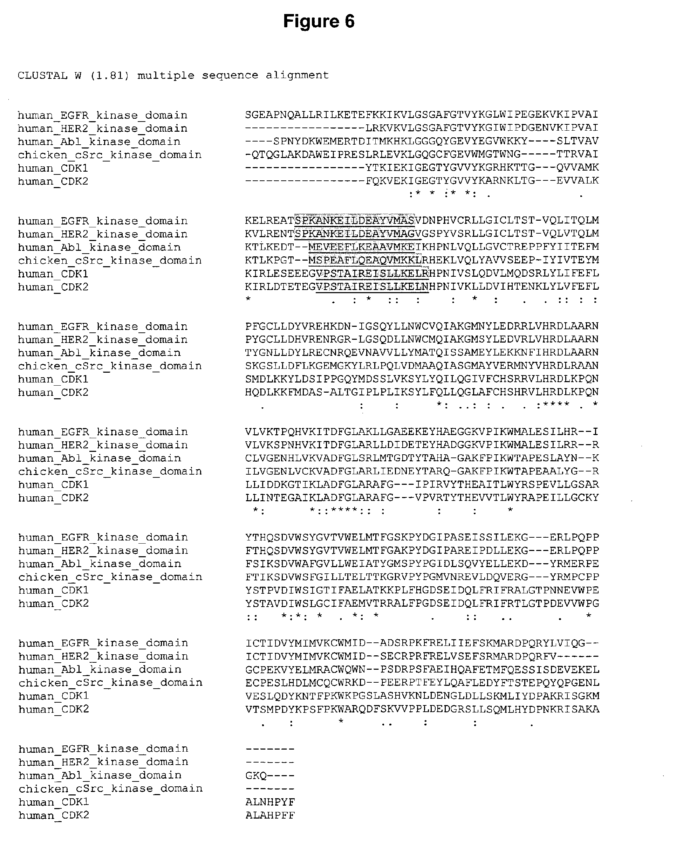

The C helix is a well-known secondary structure element occurring in protein kinases and is commonly also referred to as "subdomain III". The conformation of the residues of C helix is α helical. When going from the N-terminus to the C-terminus of a protein kinase sequence, the C helix is located between subdomain II and subdomain IV as defined in, for example, Hanks et al. (loc. cit.). In the tertiary structure, the C helix is part of the N-terminal lobe of the kinase domain and is located adjacent to the catalytic site, the activation loop and the P-loop. Accordingly, the skilled person can determine without further ado the presence and location of the C helix within any given protein kinase when provided with an experimentally determined or predicted three-dimensional structure and/or information on or prediction of the secondary structure. Moreover, availability of the amino acid sequence of any given protein kinase alone is sufficient to determine location and boundaries of the stretch of amino acid residues forming the C helix within the primary sequence. Suitable means are offered, for example, by the Pfam database of hidden Markov models (HMMs) of protein domains (Finn et al., Nucleic Acid Research (2010) Database Issue 38:D211-222). In version 24.0 of the Pfam database the protein kinase hidden Markov model is also referred to as "PF00069". Alternatively or in addition, the enclosed multiple sequence alignment of protein kinase sequences may be employed; see Figure 6. The location of the C helix is indicated in this multiple sequence alignment. Aligning the sequence of a further protein kinase to this multiple sequence alignment permits the location of the C helix in this further protein kinase sequence. A more comprehensive alignment of protein kinase sequences can be found, for example, in Hanks and Quinn, Methods in enzymology 200, 38-62, 1991. The C helix is referred to as "conserved region III" in this publication.

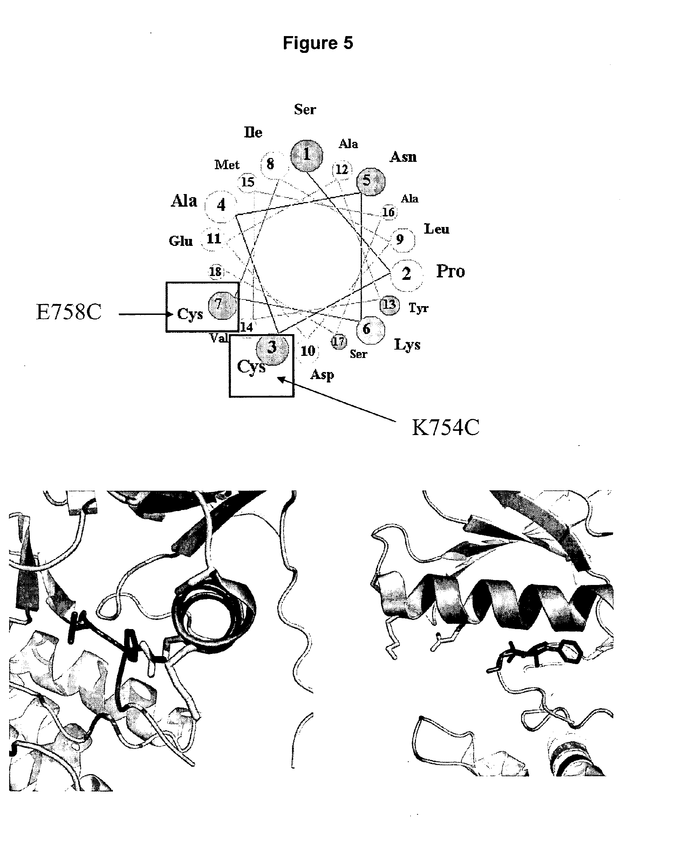

In a preferred embodiment, Glu 762 of EGFR (position 11 in the helical wheel of Figure 5; position 64 of SEQ ID NO: 1) as well as the glutamate in the corresponding position of any other protein kinase comprising a C helix and a glutamate at the aligning position, is not used for labeling, i.e., is preferred that this glutamate is not converted into an amino acid with a side chain amino group or thiol group.

The term "fluorophore" is well-known in the art and denotes a molecule or functional group within a molecule which absorbs energy such as a photon of a specific wavelength and emits energy, i.e. light at a different (but equally specific) wavelength immediately upon absorbance (unlike the case in phosphorescence) without the involvement of a chemical reaction (as the case in bioluminescence). This type of emission is known as fluorescence. Usually the wavelength of the absorbed photon is in the ultraviolet range but can reach also into the visible and infrared range. The wavelength of the emitted light is shifted towards longer wavelengths as compared to the absorption wavelength and is usually in the visible range. The amount and wavelength of the emitted energy depend primarily on the properties of the fluorophore but is also influenced by the chemical environment surrounding the fluorophore. Fluorophores can be sensitive to changes in their environment. Preferred fluorophores are sensitive to the environment. This includes changes in the polarity, charge in which occur when the conformation of the molecule to which they are attached changes. Fluorescence occurs when a molecule relaxes to its ground state after being electronically excited which, for commonly used fluorescent compounds that emit photons with energies from the UV to near infrared, typically happens in the range of between 0.5 and 20 nanoseconds. Fluorophores typically exhibit changes in intensity and/or a shift in the wavelength of maximum emission depending on the polarity of the surrounding chemical environment. In the present case, the chemical environment is formed by the structural elements of the kinase, in particular those structural elements of the kinase which are in close spatial proximity of the label, as well as by surrounding molecules including solvent molecules.

Preferred fluorophores are 6-acryloyl-2-dimethylaminonaphthalene (Acrylodan), 6-bromoacetyl-2-dimethylamino-naphthalenebadan (Badan), 2-(4'-(iodoacetamido)anilino)naphthalene-6-sulfonic acid, sodium salt (IAANS), 2-(4'-maleimidylanilino)naphthalene-6-sulfonic acid, sodium salt (MIANS), 5-((((2-iodoacetyl)amino)ethyl)amino)naphthalene-1-sulfonic acid (1,5-IAEDANS) and 5-dimethylaminonaphthalene-1-sulfonyl aziridine (dansyl aziridine) or a derivative thereof.

Other fluorophores which may be used are coumarin-based compounds, benzoxadiazole-based compounds, dapoxyl-based compounds, biocytin-based compounds, fluorescein, sulfonated rhodamine-based compounds such as AlexaFluor dyes (Molecular Probes), Atto fluorophores (Atto Technology) or Lucifer Yellow. Coumarin-based fluorophores are moderately sensitive to environment and 7-diethylamino-3-(4'-maleimidylphenyl)-4-methylcoumarin (CPM) is an example. Benzoxadiazole fluorophores are also commonly used for forming protein-fluorophore conjugates and have a strong environmental dependence with 7-fluorobenz-2-oxa-1,3-diazole-4-sulfonamide (ABD-F) and N-((2-(iodoacetoxy)ethyl)-N-methyl)amino-7-nitrobenz-2-oxa-1,3-diazole ester (IANBD) as examples. PyMPO maleimide (for thiols) or succinimide ester (for amines) and various other dapoxyl dyes have good absorptivity and exceptionally high environmental sensitivity. Examples are 1-(2-maleimidylethyl)-4-(5-(4-methoxyphenyl)oxazol-2-yl)pyridinium methanesulfonate (PyMPO-maleimide), 1-(3-(succinimidyloxycarbonyl)benzyl)-4-(5-(4-methoxyphenyl)oxazol-2-yl) pyridinium bromide (PyMPO-succinimidyl ester) and Dapoxyl (2-bromoacetamidoethyl) sulphonamide.

Recording the fluorescence emission signal at one or more wavelengths or a spectrum is usually accomplished using a fluorescence spectrometer or fluorimeter. Fluorescence spectroscopy or fluorimetry or spectrofluorimetry is a type of electromagnetic spectroscopy which analyzes fluorescence, or other emitted light, from a sample. It involves using a beam of light, usually ultraviolet light, that excites the electrons in certain molecules and causes them to emit light of a lower energy upon relaxation, typically, but not necessarily, visible light.

Two general types of instruments exist which can both be employed in the method of the invention: Filter fluorimeters use filters to isolate the incident light and fluorescent light, whereas spectrofluorimeters use diffraction grating monochromators to isolate the incident light and fluorescent light. Both types utilize the following scheme: The light from an excitation source passes through a filter or monochromator and strikes the sample. A proportion of the incident light is absorbed by the sample, and some of the molecules in the sample fluoresce. The fluorescent light is emitted in all directions. Some of this fluorescent light passes through a second filter or monochromator and reaches a detector, which is usually placed at 90° to the incident light beam to minimize the risk of transmitted or reflected incident light reaching the detector. Various light sources may be used as excitation sources, including lasers, photodiodes, and lamps; xenon and mercury vapor lamps in particular. The detector can either be single-channeled or multi-channeled. The single-channeled detector can only detect the intensity of one wavelength at a time, while the multi-channeled detects the intensity at all wavelengths simultaneously, making the emission monochromator or filter unnecessary. The different types of detectors have both advantages and disadvantages. The most versatile fluorimeters with dual monochromators and a continuous excitation light source can record both an excitation spectrum and a fluorescence spectrum. When measuring fluorescence spectra, the wavelength of the excitation light is kept constant, preferably at a wavelength of high absorption, and the emission monochromator scans the spectrum. For measuring excitation spectra, the wavelength passing though the emission filter or monochromator is kept constant and the excitation monochromator is scanning. The excitation spectrum generally is identical to the absorption spectrum as the fluorescence intensity is proportional to the absorption (for reviews see Rendell, 1987; Sharma and Schulman,1999; Gauglitz and Vo-Dinh, 2003; Lakowicz, 1999).

The term "spin label" (SL) denotes a molecule, generally an organic molecule, which possesses an unpaired electron, usually on a nitrogen atom. Spin labels are used as tools for probing proteins using electron paramagnetic resonance (EPR) spectroscopy. The site-directed spin labeling (SDSL) technique allows one to monitor the conformation and dynamics of a protein. In such examinations, amino acid-specific SLs can be used. In a further preferred embodiment, the thiol-reactive spin-label is a nitroxide radical.

A preferred method for site-specifically labeling protein sequences with a spin-label is the reaction between methanethiosulfonate spin label and cysteine, to give the spin-labeled cysteine side chain, CYS-SL:

$$MeS(O)2SSR + R'SH \longrightarrow R'SSR + MeS(O)2SH$$

where R is the nitroxide group and R'SH is a protein with a cysteine sulfhydryl, and R'SSR is the spin-labeled protein. The cysteines for labeling are placed in the desired sequence position either through solid-phase techniques or through standard recombinant DNA techniques.

Site-directed spin labeling is a technique for investigating protein local dynamics using electron spin resonance. SDSL is based on the specific reaction of spin labels carrying a reactive group with amino acids. A spin label built in protein structures can be detected by EPR spectroscopy. In SDSL, sites for attachment of spin labels such as thiol or side chain amino groups, if not naturally present, are introduced into recombinantly expressed proteins, for example by site-directed mutagenesis. In other words, by the above techniques, spin labels can be introduced into a kinase such that said kinase is specifically labeled at, preferably only at the desired position. Reactive functional groups contained within the spin label determine their specificity. At neutral pH, protein thiol groups specifically react with functional groups such as methanethiosulfonate, maleimide and iodoacetamide, creating a covalent bond with the amino acid cysteine. It is understood that the spin label according to the main embodiment no longer contains a reactive functional group. As required by the invention, the spin label (already) is connected to an amino acid of the kinase via a covalent bond. Spin labels are unique molecular reporters in that they are paramagnetic, i.e. they contain an unpaired electron. For example, nitroxide spin labels are widely used for the study of macromolecular structure and dynamics because of their stability and simple EPR signal. The nitroxyl radical (N-O) is usually incorporated into a heterocyclic ring such as pyrrolidine, and the unpaired electron is predominantly localized to the N-O bond. Once incorporated into the protein, a spin label's motions are dictated by its local environment. Because spin labels are exquisitely sensitive to motion, this has profound effects on the EPR spectrum of the spin-label attached to the protein.

The signal arising from an unpaired electron can provide information about the motion, distance, and orientation of unpaired electrons in the sample with respect to each other and to the external magnetic field. For molecules free to move in solution, EPR works on a much faster time-scale than NMR (Nuclear Magnetic Resonance) spectroscopy, and accordingly can reveal details of much faster molecular motions, i.e. nanoseconds as opposed to microseconds for NMR. The gyromagnetic ratio of the electron is orders of magnitude larger than of nuclei commonly used in NMR, and so the technique is more sensitive than NMR.

Electron paramagnetic resonance (EPR) or electron spin resonance (ESR) spectroscopy is a technique for studying chemical species that have one or more unpaired electrons, such as organic and inorganic free radicals or inorganic complexes possessing a transition metal ion. The basic physical concepts of EPR are analogous to those of nuclear magnetic resonance (NMR), but it is electron spins that are excited instead of spins of atomic nuclei. Because most stable molecules have all their electrons paired, the EPR technique is less widely used than NMR. However, this limitation to paramagnetic species also means that the EPR technique is one of great specificity, since ordinary chemical solvents and matrices do not give rise to EPR spectra.

An isotope label according to the invention is, as defined above, a moiety comprising one or more atoms which are isotopes with non-zero nuclear spin, wherein said isotopes with non-zero nuclear spin are enriched as compared to the natural occurrence thereof. Isotopes are nuclides with the same atomic number but different mass number. The most common isotopes with non-zero nuclear spin are $^1H$, $^2D$, $^{15}N$, $^{13}C$, and $^{31}p$. Preferred isotopes are $^{13}C$ and $^{15}N$. According to the present invention, two distinct types of isotope labels are envisaged. First, the isotope label, similar to fluorophores and spin labels, is a chemical moiety which is attached to a functional group of an amino acid of the protein kinase. Any moiety which is enriched with regard to isotopes having non-zero nuclear spin is envisaged. For the purpose of attaching such isotope labels, reactive groups as described above in relation to fluorophores may be used. Alternatively, the isotope label may be an amino acid residue present in the kinase, wherein the occurrence of nuclei with non-zero nuclear spin is elevated as compared to the natural occurrence in that specific residue. Preferably, all atoms of a given chemical element are replaced by a corresponding isotope with non-zero nuclear spin.

Nuclear magnetic resonance (NMR) is a physical phenomenon based upon the quantum mechanical magnetic properties of the nucleus of an atom. All nuclei that contain odd numbers of protons or neutrons have an intrinsic magnetic moment and angular momentum. The most commonly measured nuclei are hydrogen ($^1H$) (the most receptive isotope at natural abundance) and carbon ($^{13}C$), although nuclei from isotopes of many other elements (e.g. $^{113}Cd$, $^{15}N$, $^{14}N$ $^{19}F$, $^{31}P$, $^{17}O$, $^{29}Si$, $^{10}B$, $^{11}B$, $^{23}Na$, $^{35}Cl$, $^{195}Pt$) can also be observed. NMR resonant frequencies for a particular substance are directly proportional to the strength of the applied magnetic field, in accordance with the equation for the Larmor precession frequency. NMR measures magnetic nuclei by aligning them with an applied constant magnetic field and perturbing this alignment using an alternating magnetic field, those fields being orthogonal. The resulting response to the perturbing magnetic field is the phenomenon that is exploited in NMR spectroscopy and magnetic resonance imaging, which use very powerful applied magnetic fields in order to achieve high spectral resolution, details of which are described by the chemical shift and the Zeeman Effect.

The term "amino acids" has its established meaning and refers to organic molecules that have a carboxylic and an amino functional group. They are the essential building blocks of proteins.

Examples of amino acids having a free thiol group are cysteine, belonging to the 20 proteinogenic amino acids, and acetyl-cysteine being a non-standard amino acid rarely occurring in natural amino acid sequences. Proteinogenic amino acids having a free side chain amino group are lysine, histidine or arginine and amino acids being aromatic amines, such as tryptophan. Pyrrolysine, 5-hydroxylysine or o-aminotyrosine are non-standard amino acids having a free side chain amino group. The amino acids asparagine and glutamine instead contain an amide group and are not suitable in the present invention as they are not reactive to labeling agents and are thus excluded.

Tryptophan is an aromatic amino acid having an amino group in its indole ring. Aromatic amines are weak bases and thus unprotonated at pH 7. However, they can be modified using a reactive reagent such as an isothiocyanate or sulfonyl chloride.

The C helix is a structural element of protein kinases which is surface-exposed to a significant degree. As a consequence, the presence of a label in said C helix is expected to interfere not or only to an insignificant degree with the binding of compounds, more specifically inhibitors, to the labeled kinase according to the invention. Similarly, no or no significant interference with catalytic activity is expected to arise as a consequence of labeling. This is also apparent from the example enclosed herewith. Even if the catalytic activity would be modified as a consequence of labeling, this would be of no relevance for various inhibitor assay formats as described in more detail below.

If, nevertheless, a need would arise to determine to which extent labeling interferes with binding, a compound known to bind to a given kinase or determined to bind to the kinase by using the methods of screening described further below, can be contacted with the labeled kinase according to the invention on the one hand and the wild type form thereof on the other hand. To explain further, an indirect measure of binding affinity is the IC50 value for a given inhibitor. A comparison of the IC50 value for a given inhibitor determined for the labeled kinase and the wild type form thereof is indicative of any change of binding affinity arising from labeling. While it is not expected that any significant difference would occur, it is nevertheless noted that preference is given to those labeled kinases wherein the equilibrium binding constant (or IC50) between a first and a second complex differ less than by a factor of 10. The mentioned first complex is a complex comprising or consisting of a labeled kinase according to the invention and a compound known or determined to bind to said labeled kinase, and the second complex is a complex comprising or consisting of the wild type form of said kinase and the same compound. Further preferred are factors of less than 9, less than 8, less than 7, less than 6, less than 5, less than 4, less than 3, less than 2 and less than 1.5 between said two binding constants.

Even though it is not necessary that labeling does not interfere with catalytic activity, it is the subject of preferred embodiments that the catalytic activity is substantially unaltered by labeling. An aspect of the catalytic activity which is particularly easy to test for is ATPase activity. The $K_m$ for ATP may be determined for both the wild type and the labeled form. Alternatively or in addition, phosphorylation of the target of the protein kinase at issue can be determined. In any case, catalytic activity can be determined for the labeled kinase according to the invention and the wild type form thereof. In preferred embodiments, the catalytic activity differs by less than the factor of 10 between the labeled kinase according to the invention and the wild type form thereof. Further preferred are factors of less than 9, less than 8, less than 7, less than 6, less than 5, less than 4, less than 3, less than 2 and less than 1.5.

The present invention results from the unexpected discovery of the present inventors that the conformation of the C helix is sensitive to the binding of kinase inhibitors to a kinase comprising said C helix. Particular sensitivity of the C helix conformation is observed or expected upon binding of type II and type III inhibitors, these types of inhibitors defined in the background section herein above. Having said that, it is also envisaged that type I inhibitors, i.e., ATP pocket binders, may trigger conformational changes which propagate to the C helix as well. The term "conformation of the C helix" as used herein refers to the position of the C helix as an approximately rigid body within the three-dimensional structure of the protein kinase on the one hand, such as tilting, shifting or reorienting its position relative to the kinase and/or to the conformation of the C helix as such which may also be subject to certain changes in response to inhibitor binding, for example by deviating from strict α-helical conformation.

It is expected that the more an inhibitor extends beyond the ATP binding pocket, the more it will interfere with the conformation of the C helix. As a consequence, a label in the C helix is a sensitive indicator of the binding of compounds which extend beyond the ATP binding pocket. In other words, the labeled kinases according to the present invention are useful vehicles for screens, the screens being directed to the identification of compounds which bind to a kinase in the manner which extends beyond the ATP pocket. As is well-known in the art, regions outside the ATP binding pocket of a kinase are structurally conserved to a lesser degree than the ATP binding pocket itself. Using the labeled kinases according to the invention in a screening approach therefore renders the detection of specific inhibitors more likely than various conventional approaches.

In particular, inhibitors extending beyond the ATP pocket may or may not alter the conformation of the activation loop. In case the conformation of the activation loop is not altered, a kinase labeled in the C helix is an alternative sensitive vehicle for the detection of such inhibitors. A movement of the C-helix creates the space needed for such inhibitors to bind. Moreover, the new approach for screening for kinase inhibitors is expected to have the further advantages of allowing alternative chemical spaces (i.e. inhibitor scaffolds) to be explored when compared to known approaches. This is the case, because for an inhibitor to interfere with the conformation of the C helix it needs to extend into close spatial proximity of the C helix or trigger conformational changes which have an impact on the C helix. In the absence of a label in the C helix such specific effects of an inhibitor could not be detected. A further consequence of this is that inhibitors or lead compounds identified by the screens according to the present invention may have a greater likelihood of overcoming common drug resistance mutations, as is evidenced by the known EGFR inhibitor lapatinib.

As will be described in more detail below in relation to further embodiments of the invention, the labeled kinases according to the invention can be used to measure kinetics and thermodynamics of ligand binding and inhibitor binding. Moreover, screening assays using the labeled kinases according to the present invention can easily be adapted to high throughput screening methods.

In a preferred embodiment, said label in the C helix is the only fluorophore, spin label or isotope label present in said kinase. While the main embodiment allows for one or more fluorophore spin labels and/or isotope labels outside C helix, this preferred embodiment specifies that the single label present in the C helix is the only fluorophore, spin label or isotope label throughout the kinase.

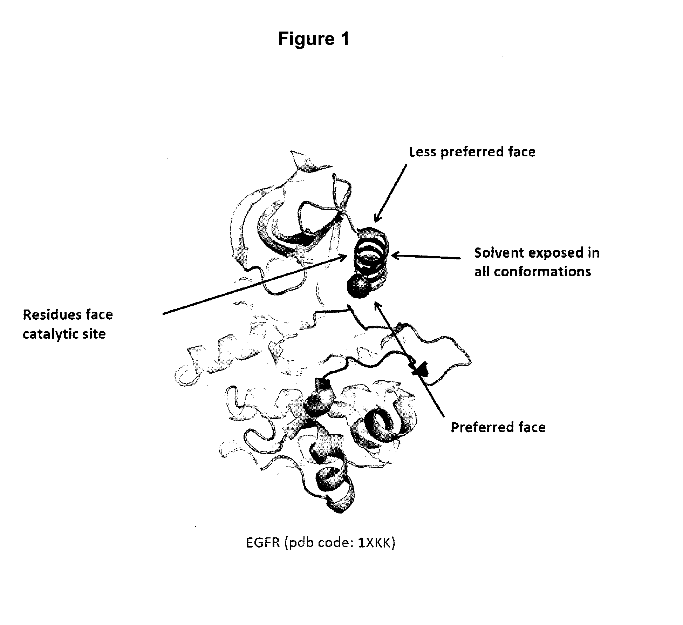

In a further preferred embodiment, the amino acid residue recited in the main embodiment faces the activation loop in the three-dimensional structure of said kinase. An exemplary structure of a protein kinase is shown in Figure 1. The drawing in Figure 1 has been prepared using the coordinates of the pdb database entry 1XKK which contains the three-dimensional structure of the kinase domain of EGFR. It is apparent that three-dimensional structures of kinases, where available, may be employed to determine those residues which face the activation loop without further ado. The face of the C helix facing the activation loop is indicated as "preferred face" in Figure 1. In the specific case displayed in Figure 1, the label is part of a residue of the preferred face; see the space-filling sphere attached to the C helix. The activation loop is the extended loop right below the C helix in Figure 1, said extended loop containing only very little ordered secondary structure. The enclosed Figure 5 provides a helical wheel representation of the C helix. In the representation chosen, position 1 is pointing away from the activation loop and position 10 is directly facing the activation loop. Accordingly, and choosing a helical wheel representation for the C helix of a protein kinase wherein position 1 is the first position pointing away from the activation loop, positions 2, 13, 6, 17, 10, 3, 14, 7 and 18 are preferred, wherein positions 13, 6, 17, 10, 3, 14 and 7 are more preferred. Boxed are residues 3 and 7 of the C helix of EGFR which both permitted successful design of a labeled kinase according to the present invention. Particularly preferred are positions 3 and 7 as well as the positions in any other protein kinase corresponding thereto. Corresponding positions in other protein kinase sequences can be determined by the skilled person without further ado, for example by making use of the multiple sequence alignments enclosed herewith (Figure 6) or described in the art as cited herein above.

In a further preferred embodiment, the amino acid sequence of the C helix of said kinase is (a) the amino acid sequence of the wild type form of said kinase; (b) differs from the amino acid sequence of said wild type form by the presence of one amino acid residue containing a thiol group or side chain amino group at a position where the amino acid sequence of the wild type does not contain such a residue; or (c) differs from the amino acid sequence of the wild type form by the absence of one or more amino acid residues containing a thiol group or side chain amino group, provided that exactly one amino acid residue or at least one amino acid residue containing a thiol group or side chain amino group is present in the C helix of said kinase.

Depending on the amino acid residues being present in the C helix of the wild type form of a given protein kinase, either the wild type form as such may be amenable to labeling, or certain sequence modifications may be required in order to introduce an amino acid residue amenable to labeling. In either case, the C helix contains at least one, preferably exactly one amino acid residue amenable to labeling, i.e., at least one or exactly one amino acid residue containing a thiol group or a side chain amino group. The recited presence of one amino acid residue according to option (b) of this embodiment may be achieved either by substitution or insertion of said one amino acid residue. Similarly, the absence of one or more amino acid residues according to option (c) may be achieved by substitution of more or more amino acid residues or deletion of one or more amino acid residues, in either case resulting in the removal of one or more amino acid residues containing a thiol group or side chain amino group. For any of these sequence modifications, means and methods are available at the skilled persons disposal. Such methods include side-directed mutagenesis as well as other recombinant synthetic or semi-synthetic techniques. In case any of the above described non-standard amino acids is to be introduced into the kinase, an amino acid stretch containing said amino acid may be chemically synthesized and then connected to the remaining part(s) of the kinase which may have been produced recombinantly or synthetically. Alternatively, kinases expressed and designed to incorporate a specialized non-standard amino acid at the desired position for subsequent labeling may be produced recombinantly by applying an altered genetic code (see e. g. Liu and Schultz, 2010). Preferred means for achieving presence of an amino acid according to option (b) are substitution, and the preferred means for achieving absence of one or more amino acid residues according to option (c) is substitution as well.

The term "delete" refers to excision of an amino acid without replacing it with another amino acid whereas the term "replace" refers to the substitution of an amino acid with another amino acid.

Preferably, amino acid replacements should be conservative. For cysteine, this means that it is preferably replaced with serine. In general, replacements of amino acids with different amino acids may be evaluated in view of whether they are conservative using the PAM250 Scoring matrix. The matrix is frequently used to score aligned peptide sequences to determine the similarity of those sequences (Pearson, 1990).

In a further preferred embodiment, (a) the amino acid sequence of said kinase outside said C helix is that of the wild type form; or (b) one, more or all amino acid residues containing a thiol group or side chain amino group and outside said C helix are deleted or replaced with amino acid residues which do not contain a thiol group or side chain amino group. While the preceding preferred embodiment relates to the sequence of the C helix, the present preferred embodiment relates to the sequence of the remainder of the kinase according to the invention. According to option (b), those amino acids which are potentially amenable to labeling (amino acid residues containing a thiol group or side chain amino group) are deleted or replaced with amino acid residues which are not amenable to labeling. This is a further preferred measure which ensures that only a single site, said single site being located in the C helix, is labeled in those cases wherein the labeled form of the kinases obtained by contacting the wild type form of the kinase in its entirety with a labeling agent under suitable conditions. As stated above, means and methods for targeted sequence modifications, including said directed mutagenesis, are available to the skilled person.

In a preferred embodiment, said amino acid residues containing a thiol group or a side chain amino group and outside the C helix according to (b) are solvent-exposed. As is known in the art, solvent-exposed amino acids, to the extent they contain suitable functional groups (thiol or side chain amino groups), are more likely to react with a reactive agent. Therefore, it is preferred that solvent-exposed amino acids, more specifically only solvent-exposed amino acids containing a thiol group or a side chain amino group and outside the C helix are deleted or replaced as defined according to (b) of the preceding embodiment.

The term "solvent-exposed" refers to the position of an amino acid in the context of the three dimensional structure of the protein of which it is a part. Amino acids buried within the protein body are completely surrounded by other amino acids and thus do not have contact with the solvent. In contrast, solvent-exposed amino acids are partially or fully exposed to the surrounding solvent and are thus accessible to chemicals potentially able to modify them. This applies e.g. to thiol- or amino-reactive labels used in the present invention which can react with solvent-exposed amino acids having a free thiol- or amino-group.

The determination of solvent-exposed amino acid residues can be performed by a skilled person without further ado. For example, the wild type form of the kinase can be contacted with agents reacting with thiol groups and or side chain amino groups. Subsequently, the labeled positions may be determined, thereby determining the solvent-exposed amino acid residues containing a thiol group or side chain amino group. Alternatively or in addition, computational means may be used. Such computational means make us of the three-dimensional structure of the protein kinase, said three-dimensional structure being either experimentally determined (such as by X-ray crystallography or NMR), or predicted by methods such as homology modeling. Provided with the coordinates of the three-dimensional structure, the skilled person may use established bioinformatic tools which determine solvent exposure of each individual atom as well as of residues in their entirety.

In a further preferred embodiment, said kinase differs from the amino acid sequence of said wild type form by the presence of exactly one amino acid residue containing a thiol group or side chain amino group at a position where the amino acid sequence of the wild type does not contain such a residue. This embodiment relates to a labeled kinase according to the present invention which has the amino acid sequence of the wild type form outside the C helix, and exactly one amino acid residue amenable to labeling, i.e., an amino acid residue containing a thiol group or a side chain amino group within the C helix, wherein said amino acid residue amenable to labeling is not present in the sequence of the C helix in the wild type form of said kinase.

In a more preferred embodiment, said exactly one amino acid residue is a cysteine replacing an amino acid residue of said wild type form.

In a further preferred embodiment, the amino acid sequence of said kinase or of the wild type form thereof is the amino acid sequence of EGFR, cSrc, Abl, HER2, or of a CDK, preferably the sequence set forth in any one of SEQ ID NOs: 2 to 12.

Further preferred kinases are described below.

The Src family of proto-oncogenic tyrosine kinases transmit integrin-dependent signals central to cell movement and proliferation. The Src family includes nine members: Src, Lck, Hck, Fyn, Blk, Lyn, Fgr, Yes, and Yrk. These kinases have been instrumental to the modern understanding of cancer as a disease with disregulated cell growth and division. The cSrc proto-oncogene codes for the cSrc tyrosine kinase. Besides its kinase domain, cSrc further comprises an SH2 domain and an SH3 domain, which act as adaptor proteins for the formation of complexes with the Src kinase domain. These domains are also involved in the auto-inhibition of the cSrc kinase domain. Mutations in this gene could be involved in the malignant progression of cancer cells. This protein specifically phosphorylates Tyr-504 residue on human leukocyte-specific protein tyrosine kinase (Lck), which acts as a negative regulatory site. It may also act on the Lyn and Fyn kinases.

Leukocyte-specific protein tyrosine kinase (Lck) is a protein that is found inside lymphocytes such as T-cells. Lck is a tyrosine kinase which phosphorylates tyrosine residues of certain proteins involved in the intracellular signaling pathways of lymphocytes. The N-terminal tail of Lck is myristoylated and palmitoylated, which tethers the protein to the plasma membrane of the cell. The protein furthermore contains an SH3 domain, an SH2 domain and in the C-terminal part the tyrosine kinase domain. The tyrosine phosphorylation cascade initiated by Lck triggers the intracellular mobilization of calcium ($Ca^{2+}$) ions and activation of important signaling cascades within the lymphocyte. These include the Ras-MEK-ERK pathway, which activates certain transcription factors such as NFAT, NFκB, and AP-1 which in turn regulate the production of a plurality of gene products, most notably, cytokines such as Interleukin-2 that promote long-term proliferation and differentiation of the activated lymphocytes. Aberrant expression of Lck has been associated with thymic tumors, T-cell leukemia and colon cancers.

The catalytic activity of the Src family of tyrosine kinases is suppressed by phosphorylation on a tyrosine residue located near the C terminus (Tyr 527 in cSrc), which is catalyzed by C-terminal Src Kinase (Csk). Given the promiscuity of most tyrosine kinases, it is remarkable that the C-terminal tails of the Src family kinases are the only known targets of Csk. Interactions between Csk and cSrc, most likely representative for Src kinases, position the C-terminal tail of cSrc at the edge of the active site of Csk. Csk cannot phosphorylate substrates that lack this docking mechanism because the conventional substrate binding site used by most tyrosine kinases to recognize substrates is destabilized in Csk by a deletion in the activation loop (Levinson, 2006).

The epidermal growth factor receptor (EGFR; ErbB-1; HER1 in humans) is the cell-surface receptor for members of the epidermal growth factor family (EGF-family) of extracellular protein ligands. The epidermal growth factor receptor is a member of the ErbB family of receptors, a subfamily of four closely related receptor tyrosine kinases: EGFR (ErbB-1), HER2/c-neu (ErbB-2), Her 3 (ErbB-3) and Her 4 (ErbB-4). Active EGFR occurs as a dimer. EGFR dimerization is induced by ligand binding to the extracellular receptor domain and stimulates its intrinsic intracellular protein-tyrosine kinase activity. As a result, autophosphorylation of several tyrosine residues in the C-terminal (intracellular) domain of EGFR occurs. This autophosphorylation elicits downstream activation and signaling by several other proteins that associate with the phosphorylated tyrosines through their own phosphotyrosine-binding SH2 domains. The kinase domain of EGFR can also cross-phosphorylate tyrosine residues of other receptors it is aggregated with, and can itself be activated in that manner. The EGFR signaling cascade activates several downstream signaling proteins which then initiate several signal transduction cascades, principally the MAPK, Akt and JNK pathways, leading to DNA synthesis and cell proliferation. Such pathways modulate phenotypes such as cell migration, adhesion, and proliferation. Mutations that lead to EGFR overexpression (known as upregulation) or overactivity have been associated with a number of cancers. Consequently, mutations of EGFR have been identified in several types of cancer, and it is the target of an expanding class of anticancer therapies.

The ABL1-protooncogene encodes a cytoplasmic and nuclear protein tyrosine kinase that has been implicated in processes of cell differentiation, cell division, cell adhesion and stress response. The activity of c-Abl protein is negatively regulated by its SH3 domain. A genetic deletion of the SH3 domain turns ABL1 into an oncogene. This genetic deletion, caused by the (9;22) gene translocation results in the head-to-tail fusion of the BCR (MIM:151410) and ABL1 genes present in many cases of chronic myelogeneous leukemia. The DNA-binding activity of the ubiquitously expressed ABL1 tyrosine kinase is regulated by CDC2-mediated phosphorylation, suggesting a cell cycle function for ABL1.

The kinases described above are preferred embodiments because all of them are involved in the development of diseases such as cancer for which at present not suitable cure is available or an improved treatment regimen is desired.

In a yet more preferred embodiment, the amino acid sequence of said kinase is the sequence set forth in SEQ ID NO: 1.

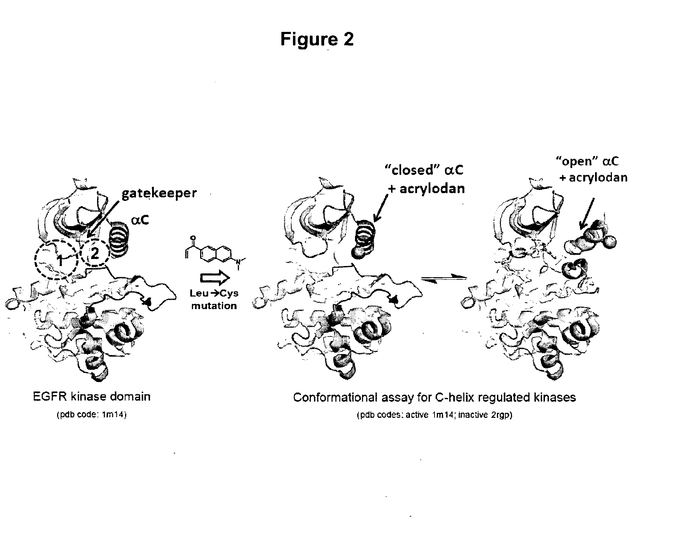

In the most preferred embodiment, there is exactly one label in said C helix or in said kinase, said label being acrylodan covalently bound to the thiol group of the cysteine residue at position 60 of SEQ ID NO: 1. The subject-matter of this last embodiment is further described in the enclosed example and shown in the attached Figure 2.

The present invention furthermore provides a method of screening for ligands of the wild type form of the kinase defined in any of the preceding embodiments, said method comprising: (a) contacting the kinase of any of the preceding embodiments with a candidate compound; and (b) recording the fluorescence emission signal, the EPR signal or the NMR signal of said label of said kinase of any of the preceding embodiments, wherein a difference in signal as compared to the signal in absence of said candidate compound is indicative of said candidate compound being a ligand of the wild type form of the kinase defined in a any of the preceding embodiments.

The term "ligand" has the meaning as established in the art. It refers to any compound which directly interacts or binds to a kinase. Preferably, the equilibrium binding constant is in the millimolar, nanomolar, picomolar or below range. It is understood that in the course of the screening typically one or more of the tested compounds is discarded because it does not exhibit any measurable binding.

Said contacting according to (a) occurs under conditions which allow binding of a ligand known to be capable of binding to said kinase. The skilled person, being acquainted with the handling of kinases as well as the design of assays using kinases as a target, can establish such suitable conditions without further ado. Such conditions include buffered solutions. Depending on whether the label present in the kinase according to the invention is a fluorophore, spin label or an isotope label, step (b) of the method according to the invention provides for recording the fluorescence emission signal, recording the EPR signal, or recording the NMR signal, respectively.

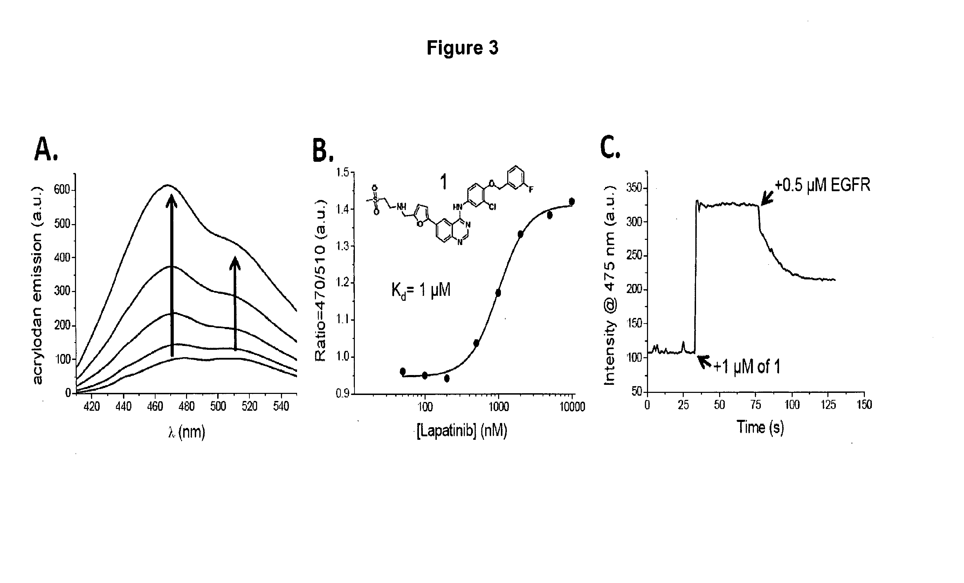

It is understood that the term "signal" embraces a measurement at a single wavelength as well as a spectral scan. Examples of a fluorescent spectrum, and how this spectrum is subjected to change upon addition of a ligand, is shown in part (a) of Figure 3 as enclosed herewith. In case for a given labeled kinase it is not yet known where in the spectrum the maximum change in signal occurs upon ligand binding, it will in general be advisable to perform a spectral scan in order to determine the wavelength of maximum change upon ligand binding. If this is already known, performing the measurement at a single wavelength, preferably at the wave length of maximum change, is sufficient. A "difference in signal" refers to a change of the entire fluorescence emission spectrum, EPR spectrum or NMR spectrum, a shift of the maximum emission wavelength, and/or a change of emission at the given wavelength.

The present invention furthermore provides a method of screening for inhibitors of the wild type form of the kinase defined above, said method comprising the method of screening for ligands as defined above and (c) determining the activity of said kinase defined herein above or of said wild type form thereof in the presence of said candidate compound, wherein a decrease in activity as compared to the absence of said candidate compound is indicative of said candidate compound being an inhibitor of the wild type form of the kinase defined above. This aspect of the present invention provides for screening for specific types of ligands which are inhibitors of kinase activity. Accordingly, the method includes the further step of monitoring the activity of the kinase. As mentioned above, the activity of the kinase may be the biochemical activity, said biochemical activity embracing ATPase activity and phosphorylating activity with regard to the target of the kinase. Alternatively or in addition, the activity at a cellular level may be determined. This includes readouts not at the level of the kinase itself, but instead, for example, of an entire pathway comprising the kinase at issue. If the kinase is disease-relevant, disease models may be used for monitoring activity. These and further kinase activity assays are within the skills of the skilled person.

Kinase inhibitors are substances capable of inhibiting the activity of kinases. They can more specifically inhibit the action of a single kinase, e.g. if they are allosteric inhibitors (Type III) or those binding to the allosteric site adjacent to the ATP-binding site and reaching into the ATP-binding pocket (Type II). Alternatively, an inhibitor can inhibit the action of a number of protein kinases, which is particularly the case if it binds exclusively to the ATP-binding pocket (Type I), which is very conserved among protein kinases.

A candidate inhibitor may belong to different classes of compounds such as small organic or inorganic molecules, proteins or peptides, nucleic acids such as DNA or RNA. Such compounds can be present in molecule libraries or designed from scratch.

Small molecules according to the present invention comprise molecules with a molecular weight of up to 2000 Da, preferably up to 1500 Da, more preferably up to 1000 Da and most preferably up to 500 Da.

Preferably, the method of screening according to the invention are effected in high-throughput format. High-throughput assays, independently of being biochemical, cellular or other assays, generally may be performed in wells of microtiter plates, wherein each plate may contain 96, 384 or 1536 wells. Handling of the plates, including incubation at temperatures other than ambient temperature, and bringing into contact with test compounds, in this case putative inhibitors, with the assay mixture is preferably effected by one or more computer-controlled robotic systems including pipetting devices. In case large libraries of test compounds are to be screened and/or screening is to be effected within short time, mixtures of, for example 10, 20, 30, 40, 50 or 100 test compounds may be added to each well. In case a well exhibits inhibitory activity, said mixture of test inhibitors may be de-convoluted to identify the one or more test inhibitors in said mixture giving rise to said activity.

Alternatively, only one test inhibitor may be added to a well, wherein each test inhibitor is applied in different concentrations. For example, the test inhibitor may be tested in two, three or four wells in different concentrations. In this initial screening, the concentrations may cover a broad range, e.g. from 10 nM to 10 $\mu$M. The initial screening serves to find hits, i.e. test inhibitors exerting inhibiting activity at at least one concentration, preferably two, more preferably all concentrations applied, wherein the hit is more promising if the concentration at which an inhibitory activity can be detected is in the lower range. This alternative serves as one preferred embodiment in accordance with the invention.

In a further aspect, the present invention provides a method of quantifying ligand or inhibitor binding to a kinase, said method comprising (a)(i) contacting a kinase according to the invention with different concentrations of a ligand or inhibitor; and/or (ii) contacting a complex, the complex comprising or consisting of a kinase according to the invention and a ligand or inhibitor, with different concentrations of unlabeled, but otherwise the same kinase or of the wild type form of said kinase; (b) recording the signal emitted by the label of said kinase according to the invention at a given time point after said contacting and for each concentration of ligand or inhibitor according to (a)(i) and/or for each concentration of unlabeled or wild type form kinase according to (a)(ii); and (c) determining the dissociation and/or association constant of said complex from the concentration dependence of said signal recorded according to (b).

This aspect of the present invention refers to so-called endpoint measurements. This is reflected by the feature "at a given time point after said contacting" as required by step (b) of the method. This method allows to determine the equilibrium dissociation constant $K_d$ of the complex formed by the kinase and the ligand. The association constant $K_a$ is related to the dissociation constant according to $K_a=1/K_d$.

The term "quantifying ligand or inhibitor binding to a kinase" refers to the determination of kinetic and/or thermodynamic parameters governing the interaction between the kinase and the ligand or inhibitor.

As previously discussed in relation to other embodiments, said contacting occurs under conditions which allow binding of a known ligand to a given kinase.

Suitable time points after said contacting can be determined by the skilled person without further ado.

The end point signal, when plotted as a function of the logarithm of the concentration according to (a)(i) or (a)(ii), usually has sigmoidal shape. The concentration corresponding to the inflection point of the sigmoidal curve corresponds to the value of $K_d$. Measuring the same samples over time and making several such curves will reveal the time needed. As the compounds bind slowly, the signal changes and the corresponding curve will shift leftward with time until it reaches its final value - this is the true $K_d$ curve for the compound at equilibrium.

In a preferred embodiment, said label is a fluorophore and said signal is the ratio of emission intensities at two different wavelengths, the two wavelengths being local maxima of the emission spectrum. This ratio provides a sensitive measure of changes in the spectrum.

This preferred embodiment is a specific implementation of the general method of quantifying ligand or inhibitor binding according to the present invention, wherein said preferred embodiment relates to those labeled kinases wherein the label is a fluorophore.

Alternatively or in addition, further parameters may be determined. These parameters include the ratio of the normalized intensity change to the average intensity change ($\Delta I_{std}$), the maximum standard intensity change ($\Delta R_{max}$) as well as the Z-factor. These quantities are defined and discussed below. A labeled kinase according to the invention, particularly a fluorescently labeled kinase according to the invention is considered particularly suitable for the screening of the kinase inhibitors if ($\Delta I_{std}$) is > 0.25, and/or ($\Delta R_{max}$) is > 0.75 and the Z-factor is > 0.5.

$\Delta I_{std}$ is the ratio of normalized intensity change to average intensity of the fluorescence emission. According to de Lorimier et al. (2002), $\Delta I_{std}$ is one of the most important criteria for characterizing a fluorescent protein conjugate as suitable for sensitive fluorescence spectroscopy. Ideally, the $\Delta I_{std}$ should have a value > 0.25 and is calculated by:

$$\Delta I_{std} = \left| \frac{2(I_1(\lambda_{std}) - I_2(\lambda_{std}))}{I_1(\lambda_{std}) + I_2(\lambda_{std})} \right|$$

where λstd = (λmax, unbound + λmax, saturated)/2 and I1, I2 are the fluorescence intensities at λstd of each spectrum respectively.

$\Delta R_{max}$ is the maximum standard intensity change of the fluorescence emission between saturated and unsaturated kinase. According to (de Lorimier et al., 2002), $\Delta R_{max}$ is another important criteria for characterizing a fluorescent protein conjugate as suitable for sensitive fluorescence spectroscopy. Ideally, the $\Delta R_{max}$ should have a value > 1.25 and is calculated by:

$$\Delta R = \left| \frac{{}^{0}A_1}{{}^{0}A_2} - \frac{{}^{\infty}A_1}{{}^{\infty}A_2} \right|$$

where ${}^{0}A1$, ${}^{0}A2$ are the areas in the absence of ligand, and ${}^{\infty}A1$, ${}^{\infty}A2$ are the areas in the presence of saturating ligand. A computer program can be used to enumerate $\Delta R$ for all possible pairs of wavelength bands in the two spectra, to identify the optimal sensing condition, defined as the maximum value of $\Delta R$.

The Z-factor is a statistical measure of the quality or power of a high-throughput screening (HTS) assay. In an HTS campaign, large numbers of single measurements of unknown samples are compared to well established positive and negative control samples to determine which, if any, of the single measurements are significantly different from the negative control. Prior to starting a large screening campaign, much work is done to assess the quality of an assay on a smaller scale, and predict if the assay would be useful in a high-throughput setting. The Z-factor predicts if useful data could be expected if the assay were scaled up to millions of samples. The Z- factor is calculated by:

$$Zfactor = 1 - \frac{3 \times (\sigma_p + \sigma_n)}{|\mu_p - \mu_n|}$$

wherein both the mean ($\mu$) and standard deviation ($\sigma$) of both the positive (p) and negative (n) controls ($\mu_p, \sigma_p, \mu_n, \sigma_n$, respectively) are taken into account.

The measurement of $\Delta I_{std}$ and $\Delta R_{max}$ as well as the determination of the Z-factor may prove useful in determining whether the label chosen is particularly suitable in the screening for inhibitors. De Lorimier discusses that the measured kinetics and $K_d$ obtained with a fluorescent tagged protein will depend on the protein, the ligand, the chosen labeling site and the fluorophore used. Therefore, the same inhibitor binding to the same labeled kinase could give different $K_d$ values depending on the label used and its position in the protein. In addition to comparing measured $K_d$ values with known values reported in literature, the determination of the above values might indicate which label should be preferred.

In a further aspect, the present invention provides a method of quantifying ligand or inhibitor binding to a kinase, said method comprising (a)(i) contacting a kinase according to the invention with at least one concentration of a ligand or inhibitor; and/or (ii) contacting a complex, the complex comprising or consisting of a kinase according to the invention and a ligand or inhibitor, with at least one concentration of unlabeled, but otherwise the same kinase or of the wild type form of said kinase; (b)recording the signal emitted by the label of said kinase according to the invention as a function of time upon said contacting according to (a); and (c)(i) determining the rate constant governing the time dependence of said signal according to (b) for (each of) the concentration(s) according to (a)(i) and determining $k_{on}$ therefrom; and/or (ii) determining the rate constant governing the time dependence of said signal according to (b) for (each of) the concentration(s) according to (a)(ii) and determining $k_{off}$ therefrom.

This aspect of the invention relates to real time measurements as indicated by the feature "as a function of time" required by step (b) of the method. The thereby obtained time dependencies allow determination of the kinetic constants $k_{on}$ and $k_{off}$ by established means which is reflected in step (c) of the method according to this aspect of the invention.

In a preferred embodiment, this method of quantifying ligand or inhibitor binding further comprises calculating the dissociation constant and/or association constant from $k_{on}$ and $k_{off}$ as determined in step (c). As is well-known in the art, the association constant $K_a$ is related to the kinetic constants $k_{on}$ and $k_{off}$ as follows: $K_a = k_{on}/k_{off}$.

In chemical kinetics, a rate constant $k$ quantifies the speed of a chemical reaction. For a chemical reaction where substance A and B are reacting to produce C, the reaction rate has the form:

$$\frac{d[C]}{dt} = k(T)[A]^m[B]^n$$

wherein k(T) is the reaction rate constant that depends on temperature, and [A] and [B] are the concentrations of substances A and B, respectively, in moles per volume of solution assuming the reaction is taking place throughout the volume of the solution. The exponents m and n are the orders and depend on the reaction mechanism. They can be determined experimentally.

A single-step reaction can also be described as:

$$\frac{d[C]}{dt} = Ae^{\frac{-E_a}{RT}}[A]^m[B]^n$$

$E_a$ is the activation energy and R is the Gas constant. Since at temperature T the molecules have energies according to a Boltzmann distribution, one can expect the proportion of collisions with energy greater than $E_a$ to vary with $e^{-Ea/RT}$. A is the pre-exponential factor or frequency factor.

$k_{on}$ and $k_{off}$ are constants that describe non-covalent equilibrium binding. When a ligand interacts with a receptor, or when a substrate interacts with an enzyme, the binding follows the law of mass action.

$$R + L \underset{k_{off}}{\overset{k_{on}}{\rightleftharpoons}} RL$$

In this equation R is the concentration of free receptor, L is the concentration of free ligand, and RL is the concentration of receptor-ligand complex. In the case of enzyme kinetics, R is the enzyme, or in this case a protein kinase, and L is the substrate, or in this case a candidate or known inhibitor. The association rate constant $k_{on}$ is expressed in units of $M^{-1}sec^{-1}$. The rate of RL formation equals [R] x [L] x $k_{on}$. The dissociation rate constant $k_{off}$ is expressed in units of $sec^{-1}$. The rate of RL dissociation equals [RL] x $k_{off}$. At equilibrium, the backward (dissociation) reaction equals the forward (association) reaction. Binding studies measure specific binding, which is a measure of [RL]. Enzyme kinetic assays assess enzyme velocity, which is proportional to [RL], the concentration of enzyme-substrate complexes.

$$RL = R \cdot L \cdot \frac{k_{on}}{k_{off}}$$

The equilibrium dissociation constant, $K_d$ is expressed in molar units and defined to equal $k_{off}/k_{on}$ to arrive at

$$RL = R \cdot L \cdot \frac{k_{on}}{k_{off}} = \frac{R \cdot L}{K_d}$$

The dissociation constant ($K_d$) corresponds to the concentration of ligand (L) at which the binding site on a particular protein is half occupied, i.e. the concentration of ligand, at which the concentration of protein with ligand bound (RL), equals the concentration of protein with no ligand bound (R). The smaller the dissociation constant, the more tightly bound the ligand is, or the higher the affinity between ligand and protein.

Accordingly, the association constant $K_a$, also called inverse $K_d$, is defined as $1/k_d$. The dissociation constant for a particular ligand-protein interaction can change significantly with solution conditions (e.g. temperature, pH and salt concentration).

In a further aspect, the present invention relates to a method of generating a kinase suitable for the screening of kinase inhibitors, said method comprising (a) deleting one or more amino acid residues having a free thiol group or free side chain amino group, if any, present in the wild type form of said kinase, the labeling of said amino acid residues not being desirable, or replacing them with amino acid residues which do not contain a free thiol or side chain amino group; (b) replacing an amino acid residue in the C helix of the kinase with an amino acid residue containing a free thiol or side chain amino group if no amino acid residue containing a free thiol or side chain amino group is or would be present in said C helix after step (a), wherein steps (a) and (b) can be effected in any order; and (c) labeling the kinase obtained from steps (a) and (b) with a thiol- or amino-reactive fluorophore, thiol- or amino-reactive spin label, and/or thiol- or amino-reactive isotope label.

Steps (a) and (b) of this method serve to ensure that labeling according to step (c) only occurs at the position where labeling is desired.

Labeling according to step (c) makes use of thiol or amino reactive forms of the fluorophore, spin label or isotope label according to the invention.

The term "thiol- or amino-reactive" denotes the property of a compound, e.g. a fluorophore, spin label or isotope label to specifically react with free thiol- or amino groups. This is due to a functional group present in said compound which directs a specific reaction with a thiol or amino group. These functional groups may be coupled to molecules such as fluorophores, spin labels or isotope labels in order to provide labels specific for free thiol- or amino-groups. Examples for thiol-specific compounds are e.g. haloalkyl compounds such as iodoacetamide, maleimides, Hg-Link™ phenylmercury compounds or TS-link™ reagents (both Invitrogen). Haloalkyl compounds react with thiol or amino groups depending on the pH.

The process of labeling involves incubation of the kinase, optionally modified in accordance with steps (a) and (b) of the method of generating a kinase, with a thiol- or amino-reactive label under mild conditions resulting in the labeling of said mutated kinase at the desired position in the C helix. Mild conditions refer to buffer pH (e.g. around pH=7 for thiol-reactive probes), ratio of label to kinase, temperature and length of the incubation step (for thiol-reactive probes e.g. 4 °C and overnight in the dark) which are known to the skilled person and provided with instruction manuals of providers of thiol- and amino-reactive probes. Such conditions may be fine-tuned in order to slow down the reaction of the chosen thiol- or amino-reactive label to ensure that labeling of said kinase is specific to the desired labeling site. In the case of fluorophore labeling, it is preferred to carry out the incubation in the dark. Increased light exposure may result in bleaching of the fluorophore and a less intense fluorescence emission. After labeling, the labeled kinase is preferably concentrated, purified by gel filtration, or washed several times with buffer to remove excess unreacted label. The wash buffer is typically the buffer used to store the labeled kinase and may also be the buffer in which the desired measurements are made.

In a preferred embodiment of the method of generating a kinase according to the invention, said amino acid residues according to step (a), the labeling of which is not desirable, are solvent-exposed. In this preferred embodiment, only those reactive amino acids are changed which are likely to react with fluorophores, spin labels or isotope labels containing a reactive group, given their exposure to solvent.

In a preferred embodiment, the method of generating a kinase according to the invention further comprises (d) contacting the kinase obtained by the method of the above method with an inhibitor of said kinase; and (e) recording the fluorescence emission signal, the EPR signal or the NMR signal of the label of the kinase; wherein a difference in the fluorescence, EPR or NMR signal is indicative of the kinase being suitable for the screening of kinase inhibitors. While such difference is expected to occur for essentially all labeled kinases according to the invention, the latter embodiment is a means to determine those labeled kinases which are particularly suitable for any of the methods disclosed herein. For example, any inhibitor screen using such particularly sensitive labeled kinase may serve to screen a compound library in particular sensitive manner.

The figures show:

**Figure 1: C helix within the protein kinase domain and possible placements of the label within the C helix.** Figure 1 uses the coordinates of the EGFR crystal structure of pdb entry 1XKK to illustrate the structural features. The structure of the EGFR kinase domain (wild type) is shown with critical structural features highlighted, including the hinge region (between upper and lower lobe) with gatekeeper residue (sticks), helix C and the activation loop (extended loop below the C helix with little ordered secondary structure). The C helix is viewed almost along the helical axis. The label, which is shown as a space-filling sphere is present at the face of the C helix which points to the activation loop.

**Figure 2: Scheme of the fluorescence-based binding assay for the C helix of EGFR.** The features of the EGFR kinase domain are depicted as described in Figure 1. Here, the ATP site (1) and subpocket beyond the gatekeeper (2) are also labeled. The wild type kinase was mutated to introduce a Leu734Cys mutation into helix C and to remove other solvent-exposed Cys residues before labeling the protein with acrylodan (sphere). The binding of an extended inhibitor in the ATP site (sticks) pushes the C helix outward into an inactive conformation and causes a change in the microenvironment of the acrylodan, thus serving as a conformational C helix assay.

**Figure 3: Detection of lapatinib binding to EGFR.** The compound was detected by monitoring the changes in emission spectra in response to increasing concentrations of lapatinib (A). Spectra revealed a robust gain in intensity at 470 nm relative to 510 nm. The $K_d$ for lapatinib binding was determined to be ~ 1 $\mu$M by plotting the ratiometric change in acrylodan emission at two wavelengths (R = 470 nm / 510 nm) against the concentration of lapatinib (B). The kinetics of lapatinib binding and dissociation were also examined by monitoring emission at a single wavelength (475 nm) over time (C). Addition of lapatinib caused a gain of fluorescence intensity while subsequent addition of a 10-fold excess of unlabeled EGFR (relative to acrylodan-labeled EGFR) induced the dissociation of lapatinib from the acrylodan-labeled EGFR and restored fluorescence to a lower level. The dissociation kinetic was slower than the binding kinetic. Addition of other known and potent ATP-competitive inhibitors of EGFR, such as dasatinib, gave no significant change in R at 1 $\mu$M.

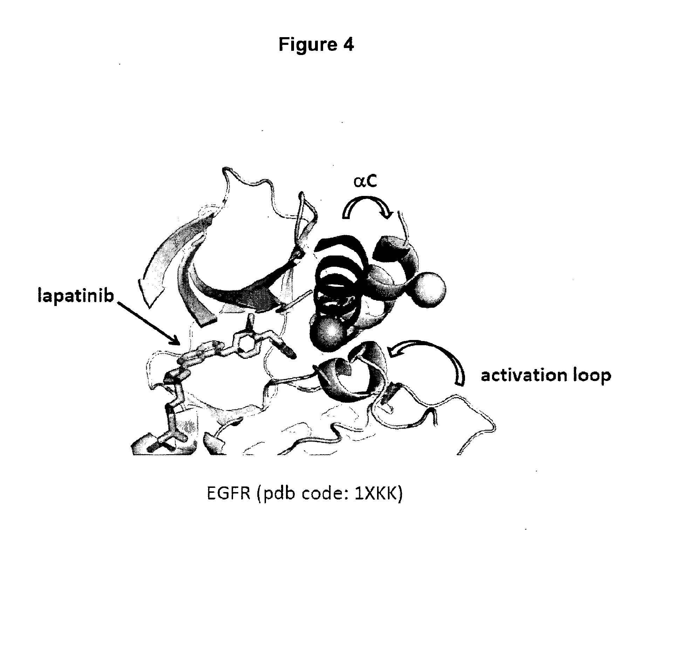

**Figure 4: The binding mode of lapatinib induces movement of the C helix of EGFR.** The structure of the EGFR kinase domain (wild type) is shown with relevant structural features highlighted as in Figure 1. Binding of lapatinib induces movement of the C-helix outward to an inactive conformation and also allows the activation loop adopt an inactive conformation. These conformational adjustments cause the fluorescence of acrylodan to change (spheres).

**Figure 5: Helical wheel representation of the C helix**. The upper part of Figure 5 provides a helical wheel representation of the C helix of EGFR. The helical wheel is representative of the C helix in general. Indicated in boxes are two positions where labels have been introduced in accordance with the present invention. In the lower part of Figure 5 a view of the C helix of EGFR is presented along the axis (left) and wherein the axis is in the paper plain (right).

**Figure 6: Multiple sequence alignment of six preferred protein kinases according to the invention.**

The examples illustrate the invention:

**Example 1: Fluorophore-labeled EGFR**

Glu 758 (position 60 of SEQ ID NO: 1) has been identified as a suitable site for labeling with the environmentally-sensitive fluorophore acrylodan, following mutation of this position into a cysteine residue. We demonstrated, using this assay, that ligands which bind in this pocket induce a change in the fluorescence emission spectrum of acrylodan. This change in fluorescence is dose-dependent and allows direct determination of $K_d$ values. In addition, the system is robust enough to also detect the binding and dissociation kinetics of ligands in real-time.

Figure 3 demonstrates the effects of inhibitor binding to fluorophore-labeled EGFR.

**Further references**

Adrian, F. J., Ding, Q., Sin, T., Velentza, A., Sloan, C., Liu, Y., Zhang, G., Hur, W., Ding, S., Manley, P., Mestan, J., Fabbro, D., Gray, N. S.. Nat Chem Biol 2006, 2, 95-102.
R. T. Aimes, W. Hemmer, S. S. Taylor. Serine-53 at the tip of the glycine-rich loop of cAMP-dependent protein kinase: role in catalysis, P-site specificity, and interaction with inhibitors. Biochemistry 2000, 39, 8325.
Annis, D. A., Nazef, N., Chuang, C. C., Scott, M. P., and Nash, H. M. (2004) A general technique to rank protein-ligand binding affinities and determine allosteric versus direct binding site competition in compound mixtures, Journal of the American Chemical Society 126, 15495-15503.
Calleja, V., Laguerre, M., Parker, P. J., Larijani, B.. PLoS Biol 2009, .7, 189-200.
R. A. Copeland, D. L. Pompliano, T. D. Meek, Nat Rev Drug Discov 2006, 5, 730.
de Lorimier, R. M., Smith, J. J., Dwyer, M. A., Looger, L. L., Sali, K. M., Paavola, C. D., Rizk, S. S., Sadigov, S., Conrad, D. W., Loew, L., and Hellinga, H. W. (2002) Construction of a fluorescent biosensor family, Protein Sci 11, 2655-2675.Dumas, J., Hatoum-Mokdad, H., Sibley, R., Riedl, B., Scott, W. J., Monahan, M. K., Lowinger, T. B., Brennan, C., Natero, R., Turner, T., Johnson, J. S., Schoenleber, R., Bhargava, A., Wilhelm, S. M., Housley, T. J., Ranges, G. E., and Shrikhande, A. (2000) 1-Phenyl-5-pyrazolyl ureas: potent and selective p38 kinase inhibitors, Bioorganic & medicinal chemistry letters 10, 2051-2054.

Fischmann, T., Smith, C., Mayhood, T., Myers, J., Reichert, P., Mannarino, A., Carr, D., Zhu, H., Wong, J., Yang, R. S., Le, H., Madison, V., Biochemistry 2009.

Gauglitz, G. and Vo-Dinh, T. (2003). Handbook of spectroscopy. Wiley-VCH.

Gschwind, A., Fischer, O. M. and Ullrich, A., The discovery of receptor tyrosine kinases: targets for cancer therapy. Nat Rev Cancer 4 (5), 361 (2004).

Hibbs, R. E., Talley, T. T., and Taylor, P. (2004) Acrylodan-conjugated cysteine side chains reveal conformational state and ligand site locations of the acetylcholine-binding protein, The Journal of biological chemistry 279, 28483-28491.

Kirkland, L. O., McInnes, C.. Biochem Pharmacol 2009.

Kluter, S., Grutter, C., Naqvi, T., Rabiller, M., Simard, J. R., Pawar, V., Getlik, M., Rauh, D. (2009). Displacement assay for the detection of stabilizers of inactive kinase conformations. J Med Chem

Knighton, D. R., Zheng, J. H., Ten Eyck, L. F., Xuong, N. H, Taylor, S. S., Sowadski, J. M. (1991). Structure of a peptide inhibitor bound to the catalytic subunit of cyclic adenosine monophosphate-dependent protein kinase. Science 253, 414-20.

Lakowicz, J. R. (1999). Principles of Fluorescence Spectroscopy. Kluwer Academic / Plenum Publishers

Levinson, N. M. et al., A Src-like inactive conformation in the abl tyrosine kinase domain. PLoS Biol 4 (5), e144 (2006).

Liu, Y. and Gray, N. S., Rational design of inhibitors that bind to inactive kinase conformations. Nat Chem Biol 2 (7), 358 (2006).

Liu, C. C. and Schultz, P. G. (2010). Adding new chemistries to the genetic code. Annu Rev Biochem 79,15.1-15.32.

B. Nagar, W. G. Bornmann, P. Pellicena, T. Schindler, D. R. Veach, W. T. Miller, B. Clarkson, J. Kuriyan, Cancer Res 2002, 62, 4236.

Pargellis, C., Tong, L., Churchill, L., Cirillo, P. F., Gilmore, T., Graham, A. G., Grob, P. M., Hickey, E. R., Moss, N., Pav, S., and Regan, J. (2002) Inhibition of p38 MAP kinase by utilizing a novel allosteric binding site, Nature structural biology 9, 268-272.

W. A Pearson, Rapid and Sensitive Sequence Comparison with FASTP and FASTA, in Methods in Enzymology, ed. R. Doolittle Academic Press, San Diego) 183(1990)63-98.

Rendell, D. (1987). Fluorescence and Phosphorescence. Crown

Richieri, G. V., Ogata, R. T., and Kleinfeld, A. M. (1999) The measurement of free fatty acid concentration with the fluorescent probe ADIFAB: a practical guide for the use of the ADIFAB probe, Molecular and cellular biochemistry 192, 87-94.M. Saraste, P. R. Sibbald, A. Wittinghofer, Trends Biochem Sci 1990, 15, 430.

Sharma, A. and Schulman, S. G. (1999). Introduction to Fluorescence Spectroscopy. Wiley interscience.Sullivan, J. E., Holdgate, G. A., Campbell, D., Timms, D., Gerhardt, S., Breed, J., Breeze, A. L., Bermingham, A., Pauptit, R. A., Norman, R. A., Embrey, K. J., Read, J., VanScyoc, W. S., and Ward, W. H. (2005) Prevention of MKK6-dependent activation by binding to p38alpha MAP kinase, Biochemistry 44, 16475-16490.

Tecle, H., Feru, F., Liu, H., Kuhn, C., Rennie, G., Morris, M., Shao, J., Cheng, A. C., Gikunju, D., Miret, J., Coli, R., Xi, S. H., Clugston, S. L., Low, S., Kazmirski, S., Ding, Y. H., Cao, Q., Johnson, T. L., Deshmukh, G. D., DiNitto, J. P., Wu, J. C.; English, J. M. (2009). The design, synthesis and potential utility of fluorescence probes that target DFG-out conformation of p38alpha for high throughput screening binding assay. Chem Biol Drug Des 74, 547-59.

Vogtherr, M., Saxena, K., Hoelder, S., Grimme, S., Betz, M., Schieborr, U., Pescatore, B., Robin, M., Delarbre, L., Langer, T., Wendt, KU., Schwalbe, H. NMR characterization of kinase p38 dynamics in free and ligand-bound forms. Angew. Chem. Int. Ed. Engl. 2006 45(6), 993-7.

Wan, P. T., Garnett, M. J., Roe, S. M., Lee, S., Niculescu-Duvaz, D., Good, V. M., Jones, C. M., Marshall, C. J., Springer, C. J., Barford, D., Marais, R., Cell 2004, 116, 855.

Yem, A. W., Epps, D. E., Mathews, W. R., Guido, D. M., Richard, K. A., Staite, N. D., and Deibel, M. R., Jr. (1992) Site-specific chemical modification of interleukin-1 beta by acrylodan at cysteine 8 and lysine 103, The Journal of biological chemistry 267, 3122-3128.

J. Zhang, P. L. Yang, N. S. Gray, Nat. Rev. Cancer 2009, 9, 28.

**Claims**

1. A kinase containing exactly one label in the C helix,

   (a) wherein said label is a fluorophore, a spin label or an isotope label,

      (a)(i) wherein said fluorophore and said spin label are covalently bound to a thiol group or side chain amino group of an amino acid residue of said C helix,
      (a)(ii) wherein said isotope label is a moiety comprising one or more atoms which are isotopes with non-zero nuclear spin, said moiety being covalently bound to a thiol group or side chain amino group of an amino acid residue of said C helix, or said isotope label is an amino acid residue of said C helix, said amino acid residue comprising one or more atoms which are isotopes with non-zero nuclear spin,

   wherein said isotopes with non-zero nuclear spin are enriched as compared to the natural occurrence thereof.

2. The kinase of claim 1, wherein said label in the C helix is the only fluorophore, spin label or isotope label present in said kinase.

3. The kinase of claim 1 or 2, wherein said amino acid residue faces the activation loop in the three-dimensional structure of said kinase.

4. The kinase of any one of claims 1 to 3, wherein the amino acid sequence of the C helix of said kinase is

   (a) the amino acid sequence of the wild type form of said kinase;
   (b) differs from the amino acid sequence of said wild type form by the presence of one amino acid residue containing a thiol group or side chain amino group at a position where the amino acid sequence of the wild type does not contain such a residue; or
   (c) differs from the amino acid sequence of the wild type form by the absence of one or more amino acid residues containing a thiol group or side chain amino group,

   provided that exactly one amino acid residue or at least one amino acid residue containing a thiol group or side chain amino group is present in the C helix of said kinase.

5. The kinase of any one of claims 1 to 4, wherein

   (a) the amino acid sequence of said kinase outside said C helix is that of the wild type form; or
   (b) one, more or all amino acid residues containing a thiol group or side chain amino group and outside said C helix are deleted or replaced with amino acid residues which do not contain a thiol group or side chain amino group, wherein preferably said amino acid residues containing a thiol group or side chain amino group and outside said C helix are solvent-exposed.

6. The kinase according to claim 5(a) to the extent claim 5(a) refers back to claims 4(b), 3, 2 and 1, wherein said kinase differs from the amino acid sequence of said wild type form by the presence of exactly one amino acid residue containing a thiol group or side chain amino group at a position where the amino acid sequence of the wild type does not contain such a residue.

7. The kinase according to claim 6, wherein said exactly one amino acid residue is a cysteine replacing an amino acid residue of said wild type form.

8. The kinase of any one of claims 1 to 7, wherein the amino acid sequence of said kinase or of the wild type form thereof is the amino acid sequence of EGFR, cSrc, Abl, HER2, or of a CDK, preferably the sequence set forth in any one of SEQ ID NOs 2 to 12.

9. The kinase of any one of claims 1 to 8, wherein the amino acid sequence of said kinase is the sequence set forth in SEQ ID NO: 1.

10. The kinase of claim 9, wherein there is exactly one label in said C helix or in said kinase, said label being acrylodan covalently bound to the thiol group of the cysteine residue at position 60 of SEQ ID NO: 1.

44

**11.** A method of screening for ligands of the wild type form of the kinase defined in any one of claims 1 to 10, said method comprising:

> (a) contacting the kinase of any one of claims 1 to 10 with a candidate compound; and
> (b) recording the fluorescence emission signal, the EPR signal or the NMR signal of said label of said kinase of any one of claims 1 to 10,

> wherein a difference in signal as compared to the signal in absence of said candidate compound is indicative of said candidate compound being a ligand of the wild type form of the kinase defined in any one of claims 1 to 10.

**12.** A method of screening for inhibitors of the wild type form of the kinase defined in any one of claims 1 to 10, said method comprising the method of claim 11 and

> (c) determining the activity of said kinase defined in any one of claims 1 to 10 or of said wild type form thereof in the presence of said candidate compound,

> wherein a decrease in activity as compared to the absence of said candidate compound is indicative of said candidate compound being an inhibitor of the wild type form of the kinase defined in any one of claims 1 to 10.

**13.** A method of quantifying ligand or inhibitor binding to a kinase, said method comprising

> (a)

> > (i) contacting a kinase according to any one of claims 1 to 10 with different concentrations of a ligand or inhibitor; and/or
> > (ii) contacting a complex, the complex comprising or consisting of a kinase according to any one of claims 1 to 10 and a ligand or inhibitor, with different concentrations of unlabeled, but otherwise the same kinase or of the wild type form of said kinase;

> (b) recording the signal emitted by the label of said kinase according to any one of claims 1 to 10 at a given time point after said contacting and for each concentration of ligand or inhibitor according to (a)(i) and/or for each concentration of unlabeled or wild type form kinase according to (a)(ii); and
> (c) determining the dissociation and/or association constant of said complex from the concentration dependence of said signal recorded according to (b).

**14.** The method according to claim 13, wherein said label is a fluorophore and said signal is the ratio of emission intensities at two different wavelengths, the two wavelengths being local maxima of the emission spectrum.

**15.** A method of quantifying ligand or inhibitor binding to a kinase, said method comprising

> (a)

> > (i) contacting a kinase according to any one of claims 1 to 10 with at least one concentration of a ligand or inhibitor; and/or
> > (ii) contacting a complex, the complex comprising or consisting of a kinase according to any one of claims 1 to 10 and a ligand or inhibitor, with at least one concentration of unlabeled, but otherwise the same kinase or of the wild type form of said kinase;

> (b) recording the signal emitted by the label of said kinase according to any one of claims 1 to 10 as a function of time upon said contacting according to (a); and
> (c)

> > (i) determining the rate constant governing the time dependence of said signal according to (b) for (each of) the concentration(s) according to (a)(i) and determining $k_{on}$ therefrom; and/or
> > (ii) determining the rate constant governing the time dependence of said signal according to (b) for (each of) the concentration(s) according to (a)(ii) and determining $k_{off}$ therefrom.

**16.** The method of claim 15, further comprising calculating the dissociation constant and/or association constant from

$k_{on}$ and $k_{off}$ as determined in step (c).

17. A method of generating a kinase suitable for the screening of kinase inhibitors, said method comprising

(a) deleting one or more amino acid residues having a free thiol group or free side chain amino group, if any, present in the wild type form of said kinase, the labeling of said amino acid residues not being desirable, or replacing them with amino acid residues which do not contain a free thiol or side chain amino group, wherein preferably said amino acid residues the labeling of which is not desirable are solvent-exposed;
(b) replacing an amino acid residue in the C helix of the kinase with an amino acid residue containing a free thiol or side chain amino group if no amino acid residue containing a free thiol or side chain amino group is or would be present in said C helix after step (a), wherein steps (a) and (b) can be effected in any order; and
(c) labeling the kinase obtained from steps (a) and (b) with a thiol- or amino-reactive fluorophore, thiol- or amino-reactive spin label, and/or thiol- or amino-reactive isotope label.

18. The method of claim 17, further comprising

(d) contacting the kinase obtained by the method of claim 17 with an inhibitor of said kinase; and
(e) recording the fluorescence emission signal, the EPR signal or the NMR signal of the label of the kinase;

wherein a difference in the fluorescence, EPR or NMR signal is indicative of the kinase being suitable for the screening of kinase inhibitors.

# Figure 1

EGFR (pdb code: 1XKK)

# Figure 2

EGFR kinase domain
(pdb code: 1m14)

Conformational assay for C-helix regulated kinases
(pdb codes: active 1m14; inactive 2rgp)

Figure 3

# Figure 4

EGFR (pdb code: 1XKK)

## Figure 5

# Figure 6

CLUSTAL W (1.81) multiple sequence alignment

```
human_EGFR_kinase_domain      SGEAPNQALLRILKETEFKKIKVLGSGAFGTVYKGLWIPEGEKVKIPVAI
human_HER2_kinase_domain      ----------------LRKVKVLGSGAFGTVYKGIWIPDGENVKIPVAI
human_Abl_kinase_domain       ----SPNYDKWEMERTDITMKHKLGGGQYGEVYEGVWKKY----SLTVAV
chicken_cSrc_kinase_domain    -QTQGLAKDAWEIPRESLRLEVKLGQGCFGEVWMGTWNG-----TTRVAI
human_CDK1                    ----------------YTKIEKIGEGTYGVVYKGRHKTTG---QVVAMK
human_CDK2                    ----------------FQKVEKIGEGTYGVVYKARNKLTG---EVVALK
                                :*  *  :*  *:  .                        .

human_EGFR_kinase_domain      KELREATSPKANKEILDEAYVMASVDNPHVCRLLGICLTST-VQLITQLM
human_HER2_kinase_domain      KVLRENTSPKANKEILDEAYVMAGVGSPYVSRLLGICLTST-VQLVTQLM
human_Abl_kinase_domain       KTLKEDT--MEVEEFLKEAAVMKEIKHPNLVQLLGVCTREPPFYIITEFM
chicken_cSrc_kinase_domain    KTLKPGT--MSPEAFLQEAQVMKKLRHEKLVQLYAVVSEEP-IYIVTEYM
human_CDK1                    KIRLESEEEGVPSTAIREISLLKELRHPNIVSLQDVLMQDSRLYLIFEFL
human_CDK2                    KIRLDTETEGVPSTAIREISLLKELNHPNIVKLLDVIHTENKLYLVFEFL
                              *              .  : *  ::  :        : *  :   . .:: : :

human_EGFR_kinase_domain      PFGCLLDYVREHKDN-IGSQYLLNWCVQIAKGMNYLEDRRLVHRDLAARN
human_HER2_kinase_domain      PYGCLLDHVRENRGR-LGSQDLLNWCMQIAKGMSYLEDVRLVHRDLAARN
human_Abl_kinase_domain       TYGNLLDYLRECNRQEVNAVVLLYMATQISSAMEYLEKKNFIHRDLAARN
chicken_cSrc_kinase_domain    SKGSLLDFLKGEMGKYLRLPQLVDMAAQIASGMAYVERMNYVHRDLRAAN
human_CDK1                    SMDLKKYLDSIPPGQYMDSSLVKSYLYQILQGIVFCHSRRVLHRDLKPQN
human_CDK2                    HQDLKKFMDAS-ALTGIPLPLIKSYLFQLLQGLAFCHSHRVLHRDLKPQN
                                .    :        :       *:  ..:  :  .   .:**** . *

human_EGFR_kinase_domain      VLVKTPQHVKITDFGLAKLLGAEEKEYHAEGGKVPIKWMALESILHR--I
human_HER2_kinase_domain      VLVKSPNHVKITDFGLARLLDIDETEYHADGGKVPIKWMALESILRR--R
human_Abl_kinase_domain       CLVGENHLVKVADFGLSRLMTGDTYTAHA-GAKFPIKWTAPESLAYN--K
chicken_cSrc_kinase_domain    ILVGENLVCKVADFGLARLIEDNEYTARQ-GAKFPIKWTAPEAALYG--R
human_CDK1                    LLIDDKGTIKLADFGLARAFG---IPIRVYTHEAITLWYRSPEVLLGSAR
human_CDK2                    LLINTEGAIKLADFGLARAFG---VPVRTYTHEVVTLWYRAPEILLGCKY
                                *:        *::****:: :       :       :       *

human_EGFR_kinase_domain      YTHQSDVWSYGVTVWELMTFGSKPYDGIPASEISSILEKG---ERLPQPP
human_HER2_kinase_domain      FTHQSDVWSYGVTVWELMTFGAKPYDGIPAREIPDLLEKG---ERLPQPP
human_Abl_kinase_domain       FSIKSDVWAFGVLLWEIATYGMSPYPGIDLSQVYELLEKD---YRMERPE
chicken_cSrc_kinase_domain    FTIKSDVWSFGILLTELTTKGRVPYPGMVNREVLDQVERG---YRMPCPP
human_CDK1                    YSTPVDIWSIGTIFAELATKKPLFHGDSEIDQLFRIFRALGTPNNEVWPE
human_CDK2                    YSTAVDIWSLGCIFAEMVTRRALFHGDSEIDQLFRIFRTLGTPDEVVWPG
                                ::   *:*:  *   . *:  *      .    ::    ..    .  *

human_EGFR_kinase_domain      ICTIDVYMIMVKCWMID--ADSRPKFRELIIEFSKMARDPQRYLVIQG--
human_HER2_kinase_domain      ICTIDVYMIMVKCWMID--SECRPRFRELVSEFSRMARDPQRFV------
human_Abl_kinase_domain       GCPEKVYELMRACWQWN--PSDRPSFAEIHQAFETMFQESSISDEVEKEL
chicken_cSrc_kinase_domain    ECPESLHDLMCQCWRKD--PEERPTFEYLQAFLEDYFTSTEPQYQPGENL
human_CDK1                    VESLQDYKNTFPKWKPGSLASHVKNLDENGLDLLSKMLIYDPAKRISGKM
human_CDK2                    VTSMPDYKPSFPKWARQDFSKVVPPLDEDGRSLLSQMLHYDPNKRISAKA
                                .  :        *      ..    :       :       .

human_EGFR_kinase_domain      -------
human_HER2_kinase_domain      -------
human_Abl_kinase_domain       GKQ----
chicken_cSrc_kinase_domain    -------
human_CDK1                    ALNHPYF
human_CDK2                    ALAHPFF
```

Europäisches
Patentamt

European
Patent Office

Office européen
des brevets

# EUROPEAN SEARCH REPORT

**Application Number**

EP 11 00 2746

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | WO 2010/009886 A1 (MAX PLANCK GESELLSCHAFT [DE]; RAUH DANIEL [DE]; SIMARD JEFFREY RAYMOND) 28 January 2010 (2010-01-28) * Abstract; paragraph bridging p. 13-14; p. 16, penultimate paragraph; p. 84, paragraph 2; claims. * | 1-18 | INV. C07B59/00 C12Q1/48 G01N33/58 |
| A | HANKS S K ET AL: "THE EUKARYOTIC PROTEIN KINASE SUPERFAMILY: KINASE (CATLYTIC) DOMAIN STRUCTURE AND CLASSIFICATION", FASEB JOURNAL, FED. OF AMERICAN SOC. FOR EXPERIMENTAL BIOLOGY, US, vol. 9, 1 May 1995 (1995-05-01), pages 576-596, XP001078836, ISSN: 0892-6638 * the whole document * | 1-18 | |
| Y | CONGREVE M ET AL: "Keynote review: Structural biology and drug discovery", DRUG DISCOVERY TODAY, ELSEVIER, RAHWAY, NJ, US, vol. 10, no. 13, 1 July 2005 (2005-07-01), pages 895-907, XP004966896, ISSN: 1359-6446, DOI: 10.1016/S1359-6446(05)03484-7 * Abstract; p. 903, RH col. first full paragraph. * | 1-18 | TECHNICAL FIELDS SEARCHED (IPC) C07B C12Q G01N |

-/--

The present search report has been drawn up for all claims

| Place of search | Date of completion of the search | Examiner |
|---|---|---|
| Munich | 1 September 2011 | López García, F |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

# EUROPEAN SEARCH REPORT

Application Number

EP 11 00 2746

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | BELLON STEVEN F ET AL: "c-Met inhibitors with novel binding mode show activity against several hereditary papillary renal cell carcinoma-related mutations", JOURNAL OF BIOLOGICAL CHEMISTRY, THE AMERICAN SOCIETY OF BIOLOGICAL CHEMISTS, INC, US, vol. 283, no. 5, 1 February 2008 (2008-02-01), pages 2675-2683, XP009111369, ISSN: 0021-9258, DOI: 10.1074/JBC.M705774200 * Abstract; in particular, penultimate sentence. * | 1-18 | |
| Y | ALAN K. KUTACH ET AL: "Crystal Structures of IL-2-inducible T cell Kinase Complexed with Inhibitors: Insights into Rational Drug Design and Activity Regulation", CHEMICAL BIOLOGY & DRUG DESIGN, vol. 76, no. 2, 1 August 2010 (2010-08-01), pages 154-163, XP55005935, ISSN: 1747-0277, DOI: 10.1111/j.1747-0285.2010.00993.x * Abstract; p. 159, RL col., paragraph 2; p. 161 "Sunitinib-induced helix C-in conformation. * | 1-18 | TECHNICAL FIELDS SEARCHED (IPC) |

-/--

The present search report has been drawn up for all claims

| Place of search | Date of completion of the search | Examiner |
|---|---|---|
| Munich | 1 September 2011 | López García, F |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

# EUROPEAN SEARCH REPORT

Application Number

EP 11 00 2746

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | ANDREAS KUGLSTATTER ET AL: "Insights into the conformational flexibility of Bruton's tyrosine kinase from multiple ligand complex structures", PROTEIN SCIENCE, vol. 20, no. 2, 1 February 2011 (2011-02-01), pages 428-436, XP55005936, ISSN: 0961-8368, DOI: 10.1002/pro.575 * Abstract; p. 433, LH col. "Compound 5 induces the helix C-out conformation of BTK"; p. 434, LH col. paragraphs 2 and 3. * | 1-18 | |
| Y | HEALY E F ET AL: "Tyrosine kinase inhibition: Ligand binding and conformational change in c-Kit and c-Abl", FEBS LETTERS, ELSEVIER, AMSTERDAM, NL, vol. 583, no. 17, 3 September 2009 (2009-09-03), pages 2899-2906, XP026587291, ISSN: 0014-5793, DOI: 10.1016/J.FEBSLET.2009.07.051 [retrieved on 2009-08-04] * Abstract; p. 2901, RH col.. * | 1-18 | TECHNICAL FIELDS SEARCHED (IPC) |
| Y | COX S ET AL: "Domain movements in protein kinases", CURRENT OPINION IN STRUCTURAL BIOLOGY, ELSEVIER LTD, GB, vol. 4, no. 6, 1 January 1994 (1994-01-01), pages 893-901, XP025581526, ISSN: 0959-440X, DOI: 10.1016/0959-440X(94)90272-0 [retrieved on 1994-01-01] * Abstract; p. 899, RH col. l. 6-9. * | 1-18 | |

The present search report has been drawn up for all claims

| Place of search | Date of completion of the search | Examiner |
|---|---|---|
| Munich | 1 September 2011 | López García, F |

EPO FORM 1503 03.82 (P04C01)

## ANNEX TO THE EUROPEAN SEARCH REPORT
## ON EUROPEAN PATENT APPLICATION NO.

EP 11 00 2746

This annex lists the patent family members relating to the patent documents cited in the above-mentioned European search report.
The members are as contained in the European Patent Office EDP file on
The European Patent Office is in no way liable for these particulars which are merely given for the purpose of information.

01-09-2011

| Patent document cited in search report | Publication date | Patent family member(s) | Publication date |
|---|---|---|---|
| WO 2010009886 A1 | 28-01-2010 | AU 2009273465 A1<br>CA 2731357 A1<br>EP 2326728 A1 | 28-01-2010<br>28-01-2010<br>01-06-2011 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **SIMARD et al.** A new screening assay for allosteric inhibitors of cSrc. *Nat Chem Bio/.,* June 2009, vol. 5 (6), 394-6
- **SIMARD et al.** Development of a fluorescent-tagged kinase assay system for the detection and characterization of allosteric kinase inhibitors. *J Am Chem Soc.,* 23 September 2009, vol. 131 (37), 13286-96
- **SIMARD et al.** High-throughput screening to identify inhibitors which stabilize inactive kinase conformations in p38alpha. *J Am Chem Soc.,* 30 December 2009, vol. 131 (51), 18478-88
- **SIMARD et al.** Fluorophore labeling of the glycine-rich loop as a method of identifying inhibitors that bind to active and inactive kinase conformations. *Am Chem Soc.,* 31 March 2010, vol. 132 (12), 4152-60
- **HANKS, STEVEN K. ; HUNTER, TONY.** The eukaryotic protein kinase superfamily: kinase (catalytic) domain structure and classification. Academic Press, 1995, vol. 9, 576-596
- **RABILLER, M. ; GETLIK, M. ; KLÜTER, S. ; RICHTERS, A. ; TÜCKMANTEL, S. ; SIMARD, J.R. ; RAUH, D.** Proteus in the world of proteins: Conformational changes in protein kinases. *Arch. Pharm. Chem. Life Sci.,* 2010, vol. 343, 193-206
- **SEROTA TAYLOR, S. ; RADZIO-ANDZELM, ELZBIETA.** Three protein kinase structures define a common motif. *Structure,* 1994, vol. 2, 345-355
- **FINN et al.** *Nucleic Acid Research,* 2010, vol. 38, D211-222
- **HANKS ; QUINN.** *Methods in enzymology,* 1991, vol. 200, 38-62
- **ADRIAN, F. J. ; DING, Q. ; SIN, T. ; VELENTZA, A. ; SLOAN, C. ; LIU, Y. ; ZHANG, G. ; HUR, W. ; DING, S. ; MANLEY, P.** *Nat Chem Biol,* 2006, vol. 2, 95-102
- **R. T. AIMES ; W. HEMMER ; S. S. TAYLOR.** Serine-53 at the tip of the glycine-rich loop of cAMP-dependent protein kinase: role in catalysis, P-site specificity, and interaction with inhibitors. *Biochemistry,* 2000, vol. 39, 8325
- **ANNIS, D. A. ; NAZEF, N. ; CHUANG, C. C. ; SCOTT, M. P. ; NASH, H. M.** A general technique to rank protein-ligand binding affinities and determine allosteric versus direct binding site competition in compound mixtures. *Journal of the American Chemical Society,* 2004, vol. 126, 15495-15503

- **CALLEJA, V. ; LAGUERRE, M. ; PARKER, P. J. ; LARIJANI, B.** *PLoS Biol,* 2009, vol. 7, 189-200
- **R. A. COPELAND ; D. L. POMPLIANO ; T. D. MEEK.** *Nat Rev Drug Discov,* 2006, vol. 5, 730
- **DE LORIMIER, R. M. ; SMITH, J. J. ; DWYER, M. A. ; LOOGER, L. L. ; SALI, K. M. ; PAAVOLA, C. D. ; RIZK, S. S. ; SADIGOV, S. ; CONRAD, D. W. ; LOEW, L.** Construction of a fluorescent biosensor family. *Protein Sci,* 2002, vol. 11, 2655-2675
- **DUMAS, J. ; HATOUM-MOKDAD, H. ; SIBLEY, R. ; RIEDL, B. ; SCOTT, W. J. ; MONAHAN, M. K. ; LOWINGER, T. B. ; BRENNAN, C. ; NATERO, R. ; TURNER, T.** 1-Phenyl-5-pyrazolyl ureas: potent and selective p38 kinase inhibitors. *Bioorganic & medicinal chemistry letters,* 2000, vol. 10, 2051-2054
- **FISCHMANN, T. ; SMITH, C. ; MAYHOOD, T. ; MYERS, J. ; REICHERT, P. ; MANNARINO, A. ; CARR, D. ; ZHU, H. ; WONG, J. ; YANG, R. S.** *Biochemistry,* 2009
- **GAUGLITZ, G. ; VO-DINH, T.** Handbook of spectroscopy. Wiley-VCH, 2003
- **GSCHWIND, A. ; FISCHER, O. M. ; ULLRICH, A.** The discovery of receptor tyrosine kinases: targets for cancer therapy. *Nat Rev Cancer,* 2004, vol. 4 (5), 361
- **HIBBS, R. E. ; TALLEY, T. T. ; TAYLOR, P.** Acrylodan-conjugated cysteine side chains reveal conformational state and ligand site locations of the acetylcholine-binding protein. *The Journal of biological chemistry,* 2004, vol. 279, 28483-28491
- **KIRKLAND, L. O. ; MCLNNES, C.** *Biochem Pharmacol,* 2009
- **KLUTER, S. ; GRUTTER, C. ; NAQVI, T. ; RABILLER, M. ; SIMARD, J. R. ; PAWAR, V. ; GETLIK, M. ; RAUH, D.** Displacement assay for the detection of stabilizers of inactive kinase conformations. *J Med Chem,* 2009
- **KNIGHTON, D. R. ; ZHENG, J. H. ; TEN EYCK, L. F. ; XUONG, N. H ; TAYLOR, S. S. ; SOWADSKI, J. M.** Structure of a peptide inhibitor bound to the catalytic subunit of cyclic adenosine monophosphate-dependent protein kinase. *Science,* 1991, vol. 253, 414-20
- **LAKOWICZ, J. R.** Principles of Fluorescence Spectroscopy. Kluwer Academic / Plenum Publishers, 1999

- **LEVINSON, N. M. et al.** A Src-like inactive conformation in the abl tyrosine kinase domain. *PLoS Biol,* 2006, vol. 4 (5), e144
- **LIU, Y. ; GRAY, N. S.** Rational design of inhibitors that bind to inactive kinase conformations. *Nat Chem Biol,* 2006, vol. 2 (7), 358
- **LIU, C. C. ; SCHULTZ, P. G.** Adding new chemistries to the genetic code. *Annu Rev Biochem,* 2010, vol. 79, 15.1-15.32
- **B. NAGAR ; W. G. BORNMANN ; P. PELLICENA ; T. SCHINDLER ; D. R. VEACH ; W. T. MILLER ; B. CLARKSON ; J. KURIYAN.** *Cancer Res,* 2002, vol. 62, 4236
- **PARGELLIS, C. ; TONG, L. ; CHURCHILL, L. ; CIRILLO, P. F. ; GILMORE, T. ; GRAHAM, A. G. ; GROB, P. M. ; HICKEY, E. R. ; MOSS, N. ; PAV, S.** Inhibition of p38 MAP kinase by utilizing a novel allosteric binding site. *Nature structural biology,* 2002, vol. 9, 268-272
- Rapid and Sensitive Sequence Comparison with FASTP and FASTA. **W. A PEARSON.** Methods in Enzymology. Academic Press, 1990, vol. 183, 63-98
- **RENDELL, D.** *Fluorescence and Phosphorescence.,* 1987
- **RICHIERI, G. V. ; OGATA, R. T. ; KLEINFELD, A. M.** The measurement of free fatty acid concentration with the fluorescent probe ADIFAB: a practical guide for the use of the ADIFAB probe. *Molecular and cellular biochemistry,* 1999, vol. 192, 87-94
- **M. SARASTE ; P. R. SIBBALD ; A. WITTINGHOFER.** *Trends Biochem Sci,* 1990, vol. 15, 430
- **SHARMA, A. ; SCHULMAN, S. G.** Introduction to Fluorescence Spectroscopy. Wiley interscience, 1999
- **SULLIVAN, J. E. ; HOLDGATE, G. A. ; CAMPBELL, D. ; TIMMS, D. ; GERHARDT, S. ; BREED, J. ; BREEZE, A. L. ; BERMINGHAM, A. ; PAUPTIT, R. A. ; NORMAN, R. A.** Prevention of MKK6-dependent activation by binding to p38alpha MAP kinase. *Biochemistry,* 2005, vol. 44, 16475-16490
- **TECLE, H. ; FERU, F. ; LIU, H. ; KUHN, C. ; RENNIE, G. ; MORRIS, M. ; SHAO, J. ; CHENG, A. C. ; GIKUNJU, D. ; MIRET, J.** The design, synthesis and potential utility of fluorescence probes that target DFG-out conformation of p38alpha for high throughput screening binding assay. *Chem Biol Drug Des,* 2009, vol. 74, 547-59
- **VOGTHERR, M. ; SAXENA, K. ; HOELDER, S. ; GRIMME, S. ; BETZ, M. ; SCHIEBORR, U. ; PESCATORE, B. ; ROBIN, M. ; DELARBRE, L. ; LANGER, T.** NMR characterization of kinase p38 dynamics in free and ligand-bound forms. *Angew. Chem. Int. Ed. Engl.,* 2006, vol. 45 (6), 993-7
- **WAN, P. T. ; GARNETT, M. J. ; ROE, S. M. ; LEE, S. ; NICULESCU-DUVAZ, D. ; GOOD, V. M. ; JONES, C. M. ; MARSHALL, C. J. ; SPRINGER, C. J. ; BARFORD, D.** *Cell,* 2004, vol. 116, 855
- **YEM, A. W. ; EPPS, D. E. ; MATHEWS, W. R. ; GUIDO, D. M. ; RICHARD, K. A. ; STAITE, N. D. ; DEIBEL, M. R., JR.** Site-specific chemical modification of interleukin-1 beta by acrylodan at cysteine 8 and lysine 103. *The Journal of biological chemistry,* 1992, vol. 267, 3122-3128
- **J. ZHANG ; P. L. YANG ; N. S. GRAY.** *Nat. Rev. Cancer,* 2009, vol. 9, 28